(19) Europäisches Patentamt
European Patent Office
Office européen des brevets

(11) EP 3 850 030 B1

(12) EUROPÄISCHE PATENTSCHRIFT

(45) Veröffentlichungstag und Bekanntmachung des Hinweises auf die Patenterteilung:
**24.04.2024 Patentblatt 2024/17**

(21) Anmeldenummer: **19765474.2**

(22) Anmeldetag: **09.09.2019**

(51) Internationale Patentklassifikation (IPC):
***C08G 59/68*** *(2006.01)*

(52) Gemeinsame Patentklassifikation (CPC):
**C08G 59/686**

(86) Internationale Anmeldenummer:
**PCT/EP2019/073919**

(87) Internationale Veröffentlichungsnummer:
**WO 2020/053114 (19.03.2020 Gazette 2020/12)**

# (54) REAKTIVER AMIN-BESCHLEUNIGER, DIESEN ENTHALTENDES REAKTIVHARZ SOWIE DEREN VERWENDUNG

REACTIVE AMINE ACCELERATOR, REACTIVE RESIN CONTAINING THE SAME AND THEIR USE

ACCÉLÉRATEUR À BASE D’AMINES RÉACTIF, RÉSINE RÉACTIVE CONTENANT LEDIT ACCÉLÉRATEUR AINSI QUE SON UTILISATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.09.2018 EP 18194457**

(43) Veröffentlichungstag der Anmeldung:
**21.07.2021 Patentblatt 2021/29**

(73) Patentinhaber: **Hilti Aktiengesellschaft**
**9494 Schaan (LI)**

(72) Erfinder:
- **GAEFKE, Gerald**
**87600 Kaufbeuren (DE)**
- **GNASS, Beate**
**86368 Gersthofen (DE)**

(74) Vertreter: **Hilti Aktiengesellschaft**
**Corporate Intellectual Property**
**Feldkircherstrasse 100**
**Postfach 333**
**9494 Schaan (LI)**

(56) Entgegenhaltungen:
**WO-A1-2012/164020**
**DE-A1- 4 336 451**
**DE-A1- 19 531 649**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 850 030 B1

## Beschreibung

Die vorliegende Erfindung betrifft einen reaktiven Amin-Beschleuniger, welcher durch Umsetzung eines primären oder sekundären aromatischen Amins mit einem Epoxid und einer α,β-ungesättigten Carbonsäure hergestellt wird, sowie dessen Verwendung in einer Reaktivharz-Zusammensetzung. Sie betrifft des Weiteren eine Reaktivharz-Zusammensetzung, insbesondere eine Epoxy(meth)acrylatharz-Zusammensetzung, mit einem erfindungsgemäßen Amin-Beschleuniger. Der erfindungsgemäße Amin-Beschleuniger wird bei der Härtung des Reaktivharzes kovalent in das Polymernetzwerk eingebaut.

## Hintergrund

Die Verwendung von chemischen Befestigungsmitteln auf Basis radikalisch härtbarer Harze ist seit langem bekannt. Im Bereich der Befestigungstechnik hat sich die Verwendung von Harzen als organisches Bindemittel für die chemische Befestigungstechnik, z.B. als Bestandteil einer Dübelmasse ("chemischer Dübel"), durchgesetzt. Es handelt sich bei solchen Dübelmassen um Verbundmassen, die als Mehrkomponenten-Systeme, üblicherweise Zweikomponenten-Systeme konfektioniert sind, wobei eine Komponente (die Reaktivharzkomponente) das radikalisch härtbare Harz und die andere Komponente (die Härterkomponente) einen Initiator (für die Radikalbildung) enthält. Andere, übliche Bestandteile, wie beispielsweise Additive, Füllstoffe, Beschleuniger, Inhibitoren, Lösungsmittel und Reaktivverdünner, können in der einen und/oder der anderen Komponente enthalten sein. Durch Vermischen der beiden Komponenten wird dann durch Radikalbildung die Härtungsreaktion, d.h. die Polymerisation, in Gang gebracht und das Harz zum Duromeren gehärtet.

Als radikalisch härtbare Harze werden in konventionellen Reaktivharzkomponenten üblicherweise unter anderem Epoxy(meth)acrylatharze, die durch Reaktion von Epoxid, z.B. Bisphenol-A-Diglycidylether (BADGE), mit α,β-ungesättigter Carbonsäure, z.B. Methacrylsäure, erhältlich sind, oder Urethan(meth)acrylatharze, die durch Reaktion von Diisocyanat, z.B. Methylendiphenylisocyanat (MDI), mit Hydroxylalkyl(meth)acrylat, z.B. Hydroxypropyl(meth)acrylat, erhältlich sind, eingesetzt. Epoxy(meth)acrylatharze bzw. Urethan(meth)acrylate werden üblicherweise mit Strahlen oder radikalisch gehärtet. Als Radikalquelle für die radikalische Härtung werden typischerweise Peroxide, wie Diacetylperoxid, Hydroperoxide oder Peroxyester zugesetzt. Aufgrund guter Lagerfähigkeit werden stabile Peroxide bevorzugt, die jedoch erst bei erhöhten Temperaturen Radikale durch thermische Zersetzung bilden. Um eine Härtung bei Raumtemperatur zu ermöglichen ist es notwendig, den Peroxidzerfall und die Radikalbildung über Additivierung zu beschleunigen, also einen sogenannten Beschleuniger zuzugeben.

Als derartige Beschleuniger werden üblicherweise Salze oder Komplexe von Übergangsmetallen (Cu, V, Co, Mn, Fe, etc.) oder tertiäre aromatische Amine als Additive in den Harzen eingesetzt. Diese Beschleuniger haben meist den Nachteil, dass sie gesundheitlich nicht unbedenklich sind oder nicht die notwendige Performance oder Lagerstabilität zeigen. Außerdem ist die kommerzielle Verfügbarkeit von als Beschleuniger geeigneten tertiären aromatischen Aminen begrenzt.

Für kennzeichnungsfreie chemische Dübel sind einige tertiäre aromatische Amine aufgrund der von ihnen ausgehenden Gesundheitsgefährdung und der damit verbundenen Kennzeichnungspflicht nicht als Beschleuniger einsetzbar.

In WO 2012/164020 A1 (DSM) wird dem Harz ein tertiäres aromatisches Amin, nämlich N,N-Diisopropanoltoluidin, welches über die Sauerstoffatome an Urethanreste gebunden ist ("UMA-gebundenes DiPpT"), als Beschleuniger zugesetzt, um die Aushärtung unter Luftzutritt zu verbessern.

Es besteht Bedarf an einem Reaktivharz, das einen Aminogruppen-haltigen Beschleuniger enthält, welcher gesundheitlich unbedenklicher als die bisher eingesetzten tertiären aromatischen Amine ist und trotzdem dem Harz die erforderliche Lagerstabilität und Performance verleiht.

## Beschreibung der Erfindung

Diese Aufgabe wird erfindungsgemäß durch die Harze gemäß Anspruch 12 und die darin verwendeten reaktiven Amin-Beschleuniger gemäß Anspruch 1 gelöst. Insbesondere zeichnet sich ein den erfindungsgemäßen Amin-Beschleuniger enthaltendes erfindungsgemäßes Reaktivharz sowie die erfindungsgemäße Reaktivharzkomponente (A), welche dieses Reaktivharz enthält, und das erfindungsgemäße Reaktivharz-System, welches diese Reaktivharzkomponente als eine Komponente umfasst, dadurch aus, dass der erfindungsgemäße Amin-Beschleuniger dank der olefinischen Gruppen bei der radikalischen Härtung nahezu vollständig im Polymernetzwerk eingebaut wird. Hierdurch wird eine Diffusion der Amin-Beschleuniger an die Oberfläche der gehärteten Werkstoffe weitgehend oder gar ganz verhindert. Ein weiterer positiver Effekt der Verwendung der hier beschriebenen reaktiven Amin-Beschleuniger als Bestandteil eines Reaktivharzes kann eine verlangsamte Sedimentationsgeschwindigkeit und somit verbesserte Lagerfähigkeit im Vergleich zu herkömmlichen Reaktivharzen sein.

Ein erfindungsgemäßer reaktiver Amin-Beschleuniger wird zwar als Additiv dem Reaktivharz zugesetzt, aber bei dessen Härtung in das ausgehärtete Harz kovalent eingebaut. Dies wird dadurch möglich, dass der erfindungsgemäße reaktive Amin-Beschleuniger synthetisiert wird durch Reaktion eines primären oder sekundären aromatischen Amins mit einem Epoxid und einer α,β-ungesättigten Carbonsäure. Der aus dieser Synthese resultierende erfindungsgemäße reaktive Amin-Beschleuniger enthält entweder eine oder zwei terminale α,β-ungesättigten Carbonsäureester. Diese reagieren dann bei der Aushärtung eines Reaktivharzes mit den anderen Monomeren, und dadurch wird der erfindungsgemäße reaktive Amin-Beschleuniger in das Harz-Rückgrat eingebaut. Dadurch ist der erfindungsgemäße reaktive Amin-Beschleuniger gesundheitlich unbedenklicher als die bisher eingesetzten tertiären Amine.

Die so hergestellte erfindungsgemäße Harzmischung (im Folgenden auch als "Reaktivharz" bezeichnet) härtet bei Raumtemperatur durch Mischen mit einem Radikalstarter wie z.B. Dibenzoylperoxid mit hoher maximaler Reaktivitätstemperatur $T_{max}$ aus, und zwar auch ohne Zusatz von weiteren Beschleunigern.

Anders als in WO 2012/164020 A1 bildet sich die tertiäre Struktur des Amin-Beschleunigers in einem erfindungsgemäßen Harz bei der Reaktion eines primären oder sekundären aromatischen Amins mit einem Epoxid und einer α,β-ungesättigten Carbonsäure. Hierdurch sind z.B. auch unsymmetrische Strukturen und Strukturen ohne Kettenverlängerung über sekundäre Amine möglich.

Der erfindungsgemäße Amin-Beschleuniger - im folgenden auch "reaktiver Amin-Beschleuniger" genannt und in den Ansprüchen auch als "Beschleuniger" bezeichnet - entsteht durch Umsetzung eines primären oder sekundären aromatischen Amins mit einem Diglycidylether und einer α,β-ungesättigten Carbonsäure. In dieser Synthese wird (1) ein aromatisches primäres oder sekundäres Amin umgesetzt mit (2) einem Diglycidylether der im folgenden Reaktionsschema abgebildeten Formel und (3) einer α,β-ungesättigten Carbonsäure. Die Reaktion erfolgt typischerweise in Gegenwart von (4) einem Katalysator. Optional kann (5) ein Inhibitor im Reaktionsgemisch vorhanden sein. Eine schematische Darstellung der Reaktion ist wie folgt:

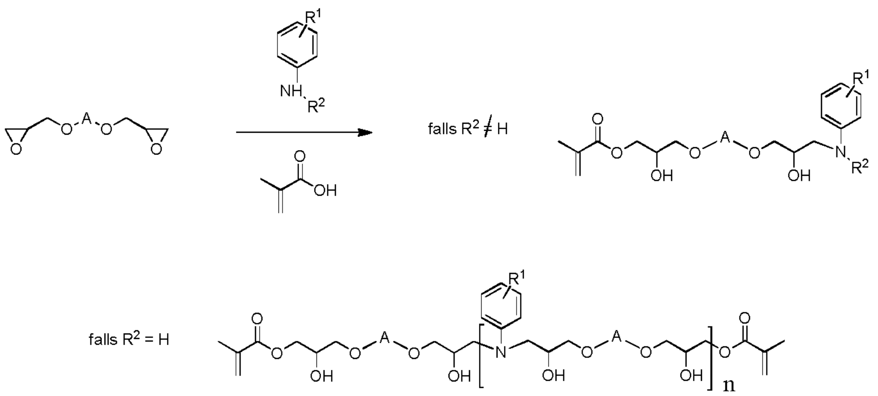


Der Phenylring steht hier als Platzhalter für einen Aromatenrest. In einer bevorzugten Ausführungsform ist dieser Aromatenrest ein Phenylring oder Naphthylring, bevorzugter ein Phenylring. Die Bedeutung der Platzhalter A, $R^1$, $R^2$ und n wird weiter unten beschrieben.

Eine exemplarische erfindungsgemäße Synthese mit einem primären aromatischen Amin (hier: para-Toluidin) erfolgt wie folgt:

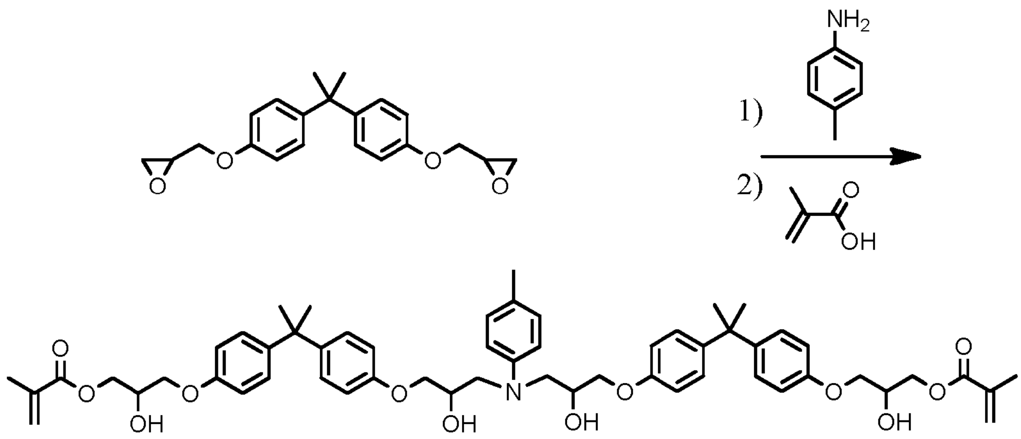


**DiPpT** 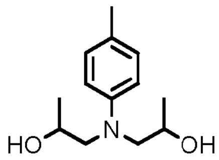

Das hier zum Vergleich ebenfalls abgebildete Di-iso-propanol-p-toluidin (DiPpT) ist ein typisches tertiäres Amin, wie es im Stand der Technik als Beschleuniger eingesetzt wird und wie es z.B. in WO 12/164020 A1 als Teil des dort beschriebenen UMA-gebundenen DiPpT verwendet wird.

Für die erfindungsgemäße Synthese des erfindungsgemäßen Amin-Beschleunigers werden die Ausgangsstoffe bevorzugt ausgewählt aus den folgenden Gruppen:

1) Das aromatische primäre oder sekundäre Amin ist ausgewählt aus der Gruppe von aromatischen primären oder sekundären Aminen, in welchen der Aromatenrest entweder unsubstituiert ist, oder substituiert ist mit einem oder mehreren Substituenten $R^1$ ausgewählt aus der Gruppe bestehend aus Halogen, Pseudohalogen, $C_1$-$C_{20}$-Alkyl, Hydroxy-$C_1$-$C_{20}$-Alkyl, $C_2$-$C_{20}$-Alkenyl, Hydroxy-$C_2$-$C_{20}$-Alkenyl, $C_2$-$C_{20}$-Alkinyl, Hydroxy-$C_2$-$C_{20}$-Alkinyl und Phenyl. $R^1$ ist bevorzugt ausgewählt aus der Gruppe bestehend aus Halogen, Hydroxy-$C_1$-$C_{20}$-Alkyl und $C_1$-$C_{20}$-Alkyl. Besonders bevorzugt ist $R^1$ ausgewählt aus der Gruppe bestehend aus Halogen und $C_1$-$C_{20}$-Alkyl. Ganz besonders bevorzugt ist $R^1$ ausgewählt aus der Gruppe bestehend aus Chlor, Brom und $C_1$-$C_6$-Alkyl, insbesondere aus der Gruppe bestehend aus Chlor, Brom und $C_1$-$C_4$-Alkyl.

Der Aromatenrest des aromatischen primären oder sekundären Amins ist substituiert mit keinem, einem oder mehreren Substituenten $R^1$. Bevorzugt ist der Aromatenrest des aromatischen primären Amins substituiert mit einem, zweien oder dreien Substituenten $R^1$, bevorzugter mit einem oder zweien Substituenten $R^1$. Bevorzugt ist der Aromatenrest des aromatischen sekundären Amins substituiert mit keinem, einem, zweien oder dreien Substituenten $R^1$, bevorzugter mit keinem, einem oder zweien Substituenten $R^1$, noch bevorzugter mit keinem oder einem Substituenten $R^1$.

Der Aromatenrest im aromatischen primären oder sekundären Amin ist ein Phenylrest oder Naphthylrest, besonders bevorzugt ein Phenylrest.

In einer bevorzugten Ausführungsform ist das aromatische primäre Amin ein Alkylanilin, das heißt, es hat einen Phenylring als Aromatenrest, und dieser trägt ein $R^1$, welches eine Alkylgruppe ist. Bevorzugt ist dieses $R^1$ ein $C_1$-$C_4$-Alkyl, noch bevorzugter ist es Methyl und das Alkylanilin somit Toluidin. Daneben können noch weitere Substituenten $R^1$ ausgewählt aus den oben für $R^1$ angegebenen Gruppen vorhanden sein. In einer bevorzugten Ausführungsform ist kein weiteres $R^1$ vorhanden. In einer weiteren bevorzugten Ausführungsform sind ein oder zwei weitere $R^1$ vorhanden, noch bevorzugter ist nur ein weiteres $R^1$ vorhanden. Das weitere $R^1$ ist in einer bevorzugten Ausführungsform ausgewählt aus der Gruppe bestehend aus Halogen und $C_1$-$C_{20}$-Alkyl. Ganz besonders bevorzugt ist $R^1$ ausgewählt aus der Gruppe bestehend aus Chlor, Brom und $C_1$-$C_6$-Alkyl, insbesondere aus der Gruppe bestehend aus Chlor, Brom und $C_1$-$C_4$-Alkyl, und ganz besonders aus der Gruppe bestehend aus Chlor und Brom. In einer bevorzugten Ausführungsform ist das aromatische primäre Amin ein am Aromaten halogeniertes Toluidin ohne weiteren Substituenten $R^1$, oder ein am Aromaten halogeniertes Toluidin, welches am Aromaten eine weitere $C_1$-$C_4$-Alkylgruppe, bevorzugt eine weitere Methylgruppe, trägt. Ein am Aromaten halogeniertes Toluidin ohne weiteren Substituenten $R^1$ ist besonders bevorzugt.

In einer bevorzugten Ausführungsform ist das aromatische sekundäre Amin ein Anilin oder ein Alkylanilin, das heißt, es hat einen Phenylring als Aromatenrest, und dieser trägt entweder kein $R^1$ oder ein $R^1$, welches eine Alkylgruppe ist. In einer Ausführungsform ist das aromatische sekundäre Amin ein Anilin. In einer anderen Ausführungsform ist das

aromatische sekundäre Amin ein Alkylanilin, das heißt, es trägt ein $R^1$, welches eine Alkylgruppe ist. Bevorzugt ist dieses $R^1$ ein $C_1$-$C_4$-Alkyl, noch bevorzugter ist es Methyl und das Alkylanilin somit Toluidin. Daneben können noch weitere Substituenten $R^1$ ausgewählt aus den oben für $R^1$ angegebenen Gruppen vorhanden sein. In einer bevorzugten Ausführungsform ist kein weiteres $R^1$ vorhanden. In einer weiteren bevorzugten Ausführungsform sind ein oder zwei weitere $R^1$ vorhanden, noch bevorzugter ist nur ein weiteres $R^1$ vorhanden. Das weitere $R^1$ ist in einer bevorzugten Ausführungsform ausgewählt aus der Gruppe bestehend aus Halogen und $C_1$-$C_{20}$-Alkyl. Ganz besonders bevorzugt ist das weitere $R^1$ ausgewählt aus der Gruppe bestehend aus Chlor, Brom und $C_1$-$C_6$-Alkyl, insbesondere aus der Gruppe bestehend aus Chlor, Brom und $C_1$-$C_4$-Alkyl, und ganz besonders aus der Gruppe bestehend aus Chlor und Brom. In einer bevorzugten Ausführungsform ist das aromatische sekundäre Amin ein am Aromaten halogeniertes Toluidin oder Anilin ohne weiteren Substituenten $R^1$, oder ein am Aromaten halogeniertes Toluidin oder Anilin, welches am Aromaten eine weitere $C_1$-$C_4$-Alkylgruppe, bevorzugt eine weitere Methylgruppe, trägt. Ein am Aromaten halogeniertes Toluidin oder Anilin ohne weiteren Substituenten $R^1$ ist besonders bevorzugt.

Ist nur ein $R^1$ vorhanden, so befindet es sich in Relation zur Aminogruppe bevorzugt in *meta*- oder *para*-Position. Dasselbe gilt beim Vorhandensein von mehreren $R^1$. Sind zwei $R^1$ vorhanden, so befindet sich bevorzugt eines davon in *meta*-Position und das andere in *para*-Position. Sind drei $R^1$ vorhanden, so befindet sich bevorzugt mindestens eines davon in *meta*-Position und eines in *para*-Position.

In den sekundären Aminen ist der Substituent $R^2$ am Stickstoff, welcher neben dem am Stickstoff gebundenen Aromaten vorhanden ist, bevorzugt ausgewählt aus der Gruppe bestehend aus $C_1$-$C_{20}$-Alkyl, Hydroxy-$C_1$-$C_{20}$-Alkyl, $C_2$-$C_{20}$-Alkenyl, Hydroxy-$C_2$-$C_{20}$-Alkenyl, $C_2$-$C_{20}$-Alkinyl, Hydroxy-$C_2$-$C_{20}$-Alkinyl. $R^2$ ist bevorzugt ausgewählt aus der Gruppe bestehend aus Hydroxy-$C_1$-$C_{20}$-Alkyl und $C_1$-$C_{20}$-Alkyl. Besonders bevorzugt ist $R^2$ ausgewählt aus der Gruppe bestehend aus Hydroxy-$C_1$-$C_{12}$-Alkyl und $C_1$-$C_{12}$-Alkyl. Ganz besonders bevorzugt ist $R^2$ ausgewählt aus der Gruppe bestehend aus Hydroxy-$C_1$-$C_4$-Alkyl und $C_1$-$C_4$-Alkyl.

2) Der Diglycidylether ist bevorzugt ausgewählt aus der Gruppe bestehend aus Diglycidylethern von Diolen von Kohlenwasserstoffen mit 2 bis 20 C-Atomen, bevorzugt mit 4 bis 15 C-Atomen. Die Kohlenwasserstoffe können verzweigt oder unverzweigt sein. Die Kohlenwasserstoffe können aromatisch oder aliphatisch oder eine Kombination davon sein. Die Diole sind bevorzugt ausgewählt aus der Gruppe bestehend aus Bisphenolen, insbesondere Bisphenol A, Bisphenol F, und Bisphenol S, Neopentylglycol, Ethylenglycol, Phenol-Novolac-Harz, Cresol-Novolac-Harz, und 1,4-Butandiol. Bevorzugt ist der Diglycidylether ausgewählt aus der Gruppe bestehend aus Diglycidylether von Bisphenol A, Diglycidylether von Bisphenol F, und Diglycidylether von Bisphenol S. Noch bevorzugter ist der Diglycidylether der Diglycidylether von Bisphenol A. Es ist auch möglich, Oligo- oder Polymere Diole zu verwenden.

Bei der Verwendung von sekundären Aminen ist es auch möglich anstelle eines Glycidylethers aus einem Diol einen Glycidylether aus einem Poylol, d.h. eine Verbindung mit mehr als zwei Hydroxylgruppen, insbesondere einem Triol und einem Tetraol, also einer Verbindung mit drei bzw. vier Hydroxylgruppen, zu verwenden, da hierdurch die Gefahr der Bildung hochviskoser polymerer Verbindungen nicht gegeben ist.

3) Die $\alpha,\beta$-ungesättigte Carbonsäure ist bevorzugt ausgewählt aus der Gruppe bestehend aus verzweigten und unverzweigten $C_2$-$C_{10}$-$\alpha,\beta$-ungesättigten Carbonsäuren, bevorzugter aus der Gruppe bestehend aus verzweigten und unverzweigten $C_2$-$C_6$-$\alpha,\beta$-ungesättigten Carbonsäuren. Besonders bevorzugt ist sie ausgewählt aus der Gruppe bestehend aus Tiglinsäure ((E)-2,3-Dimethylacrylsäure), Sorbinsäure (Hexadiensäure), Crotonsäure (*trans*-Butensäure), Methacrylsäure und Acrylsäure. Bevorzugter ist die $\alpha,\beta$-ungesättigte Carbonsäure ausgewählt aus der Gruppe bestehend aus Methacrylsäure und Acrylsäure. Noch bevorzugter ist sie Methacrylsäure.

4) Der Katalysator kann jeder konventionell für die Katalyse der Reaktion eines Epoxids mit einer $\alpha,\beta$-ungesättigten Carbonsäure zum entsprechenden Carbonsäureester verwendete Katalysator sein. Bevorzugt ist der Katalysator ein Tetraalkylammoniumhalogenid, bevorzugter ausgewählt aus der Gruppe bestehend aus Tetraalkylammoniumbromid und Tetraalkylammoniumchlorid. Bevorzugter ist der Katalysator ausgewählt aus der Gruppe bestehend aus Tetraethylammoniumbromid, Tetrabutylammoniumbromid, Tetraethylammoniumchlorid und Tetrabutylammoniumchlorid. Besonders bevorzugt ist der Katalysator ein in den Beispielen verwendeter Katalysator.

5) Der optional und bevorzugt tatsächlich verwendete Inhibitor kann jeder herkömmlich in der Synthese von Epoxy(meth)acrylatharzen verwendete Inhibitor sein. Geeignete Inhibitoren werden weiter unten genauer beschrieben.

Das Herstellungsverfahren für einen erfindungsgemäßen reaktiven Amin-Beschleuniger und für ein diesen enthaltendes erfindungsgemäßes Reaktivharz verläuft typischerweise wie folgt:

1. Herstellung reaktiver Amin-Beschleuniger

Ein Diglycidylether (2) wird mit einer $\alpha,\beta$-ungesättigten Carbonsäure, beispielsweise mit (Meth)acrylsäure (3)

in Anwesenheit eines Katalysators (4) und von einem oder mehreren primären oder sekundären aromatischen Amin(en) (1) zur Reaktion gebracht (typischerweise bei einer Temperatur von 80°C bis 120°C). Optional (und bevorzugt) enthält die Reaktionsmischung auch einen oder mehrere Inhibitoren (5). Die Reaktionsmischung enthält darüber hinaus bevorzugt keine weiteren Zutaten. Bei dieser Reaktion entsteht der erfindungsgemäße reaktive Amin-Beschleuniger. Exemplarische Reaktionen sind in den Beispielen beschrieben. Wenn ein sekundäres Amin verwendet wird, kann auch ein höherwertiger Glycidylether verwendet werden.

Um bei Verwendung von primären Anilinen wie beispielsweise *para*-Toluidin die Viskosität zu erniedrigen, kann vor Zugabe des aromatischen Amins der Diglycidylether partiell mit einem Teil der $\alpha,\beta$-ungesättigten Carbonsäure (beispielsweis (Meth)acrylsäure) umgesetzt werden. Für *para*-Toluidin ist dies exemplarisch in den Beispielen beschrieben.

Das Äquivalent-Verhältnis Diglycidylether : $\alpha,\beta$-ungesättigte Carbonsäure : primäres aromatisches Amin ist typischerweise im Bereich von 1 : 0,3 : 0,8 bis 1 : 2,09 : 0,01, bevorzugt von 1:0,6:0,7 bis 1:2,05:0,05, besonders bevorzugt etwa 1 : 1,1 : 0,5.

Das Äquivalent-Verhältnis Diglycidylether : $\alpha,\beta$-ungesättigte Carbonsäure : sekundäres aromatisches Amin ist typischerweise im Bereich von 1 : 0,1 : 2 bis 1 : 2,09 : 0,01, bevorzugt von 1:0,5:1,6 bis 1:2,05:0,05, besonders bevorzugt etwa 1 : 1,1 : 1.

Wenn eine Mischung aus primärem und sekundärem Amin verwendet wird, liegt der Äquivalent-Wert für die Mischung aus primärem und sekundärem aromatischen Amin typischerweise in einem Bereich zwischen dem Äquivalent-Wert für das sekundäre Amin (Untergrenze) und dem Äquivalent-Wert für das primäre Amin (Obergrenze). Der Wert für die Mischung ergibt sich dabei aus dem molaren Verhältnis zwischen primärem und sekundärem Amin.

Das nach Ende der Reaktion erhaltene Reaktionsgemisch wird nicht weiter aufgearbeitet, d.h. der reaktive Amin-Beschleuniger wird nicht isoliert. Optional wird zu dem Reaktionsgemisch nach Abschluss der Reaktion zu dem reaktiven Amin-Beschleuniger noch ein oder mehrere Inhibitoren und/oder ein oder mehrere Reaktivverdünner gegeben.

2. Herstellung Backbone-Harz/Reaktivharz-Masterbatch

Ein Diglycidylether, beispielsweise Bisphenol-A-diglycidylether, und eine $\alpha,\beta$-ungesättigte Carbonsäure, beispielsweise Methacrylsäure, werden in Anwesenheit eines Katalysators und eines Inhibitors (dient dazu, das durch die Polymerisation gebildete Backbone-Harz zu stabilisieren) zur Reaktion gebracht. Hierbei entsteht das Backbone-Harz.

Das nach Ende der Reaktion erhaltene Reaktionsgemisch wird als Reaktivharz-Masterbatch bezeichnet. Dieses wird nicht weiter aufgearbeitet, d.h. das Backbone-Harz wird nicht isoliert.

3. Herstellung Reaktivharz

Das Reaktionsgemisch aus der Herstellung des reaktiven Amin-Beschleunigers wird versetzt mit einem Backbone-Harz bzw. Reaktivharz-Masterbatch, beispielsweise dem Reaktionsgemisch aus der unter 2. beschriebenen Herstellung Backbone-Harz/Reaktivharz-Masterbatch, ein oder mehreren Reaktivverdünnern und ein oder mehreren Inhibitoren.

Optional werden zwei oder mehr reaktive Amin-Beschleuniger verwendet.

Die Reihenfolge, in welcher die einzelnen Komponenten des Reaktivharzes miteinander vermischt werden, ist nicht relevant. Typischerweise wird der reaktive Amin-Beschleuniger vorgelegt, und dann werden nacheinander der Reaktivharz-Masterbatch, der Reaktivverdünner, und der Inhibitor zugesetzt.

Typischerweise werden der reaktive Amin-Beschleuniger und das Backbone-Harz bzw. der Reaktivharz-Masterbatch getrennt voneinander hergestellt und zur Herstellung des Reaktivharzes miteinander vermischt. Dies gilt insbesondere für die Herstellung von Urethan(meth)acrylat-basierten Reaktivharzen. In einer weiteren Ausführungsform wird jedoch das Reaktivharz dadurch hergestellt, dass zunächst der reaktive Amin-Beschleuniger und dann das Backbone-Harz hintereinander im selben Gefäß hergestellt werden, dass also eine mehrstufige Eintopfreaktion durchgeführt wird. Dies gilt insbesondere für die Herstellung von Epoxy(meth)acrylat-basierten Reaktivharzen. In einer weiteren Ausführungsform wird das Reaktivharz dadurch hergestellt, dass zunächst der reaktive Amin-Beschleuniger und ein Teil des Backbone-Harzes in einer einstufigen Eintopfreaktion hergestellt und dann der weitere Teil des Backbone-Harzes nachgeschaltet im selben Gefäß hergestellt wird. Dies gilt ebenfalls für die Herstellung von Epoxy(meth)acrylat-basierten Reaktivharzen. In einer noch weiteren Ausführungsform werden einzelne Komponenten des Reaktivharzes, insbesondere thermisch stabile und nicht reaktive Komponenten, der Reaktionsmischung zur Herstellung des reaktiven Amin-Beschleunigers bereits vor Beginn der Reaktion zum reaktiven Amin-Beschleuniger zugesetzt.

Hierdurch erhält man das Epoxy(meth)acrylat- bzw. Urethan(meth)acrylat-Reaktivharz mit dem erfindungsgemäßen reaktiven Amin-Beschleuniger.

Ein erster Gegenstand der Erfindung ist ein reaktiver Amin-Beschleuniger, insbesondere ein Beschleuniger mit der generischen Formel (I) oder (II), hergestellt durch das hier beschriebene Herstellungsverfahren, also indem (1)

ein aromatisches primäres oder sekundäres Amin, oder eine Mischung aus zweien oder mehreren davon, umgesetzt wird mit (2) einem Diglycidylether eines Diols mit 2 bis 30 C-Atomen, und (3) einer $\alpha,\beta$-ungesättigten Carbonsäure, vorzugsweise einer $\alpha,\beta$-ungesättigten Carbonsäure ausgewählt aus der Gruppe bestehend aus Tiglinsäure, Sorbinsäure, Crotonsäure, Methacrylsäure und Acrylsäure. Die Reaktion erfolgt typischerweise in Gegenwart von (4) einem Katalysator. Optional kann (5) ein Inhibitor im Reaktionsgemisch vorhanden sein. Hierbei führt die Öffnung der im Diglycidylether vorhanden Epoxidgruppen zur Ausbildung von Glycerin-Brücken zwischen dem Amin und dem Diol und zwischen der $\alpha,\beta$-ungesättigten Carbonsäure und dem Diol. Ein solchermaßen hergestellter Amin-Beschleuniger kann auch eine Mischung aus verschiedenen Verbindungen, die bei dieser Reaktion entstehen, sein.

Ein zweiter Gegenstand der Erfindung ist ein diesen reaktiven Amin-Beschleuniger enthaltendes Reaktivharz.

Ein dritter Gegenstand ist eine Reaktivharzkomponente (A), die ein erfindungsgemäßes Reaktivharz enthält.

Ein vierter Gegenstand ist ein Reaktivharz-System mit einer erfindungsgemäßen Reaktivharzkomponente (A) und einer Härterkomponente (B), die einen Initiator (wie etwa ein Peroxid) für die Härtung des in dem Reaktivharz enthaltenen Backbone-Harzes enthält. Die Komponenten (A) und (B) sind bis zur Verwendung des Reaktivharz-Systems räumlich getrennt voneinander verpackt, damit erst eine Reaktion stattfindet, wenn die beiden Komponenten miteinander in Kontakt gebracht werden.

Ein fünfter Gegenstand der Erfindung ist ein Verfahren zur Herstellung des erfindungsgemäßen reaktiven Amin-Beschleunigers.

Ein sechster Gegenstand der Erfindung ist die Verwendung eines erfindungsgemäßen reaktiven Amin-Beschleunigers, insbesondere einer Verbindung mit der generischen Formel (I) oder (II), als Beschleuniger in einem Reaktivharz.

Ein siebter Gegenstand der Erfindung ist die Verwendung eines erfindungsgemäßen Reaktivharz-Systems zur chemischen Befestigung von Verankerungsmitteln in Bohrlöchern oder zum baulichen Kleben.

**Generische Formeln von erfindungsgemäßen reaktiven Amin-Beschleunigern:**

Ein erfindungsgemäßer reaktiver Amin-Beschleuniger, der mit einem primären aromatischen Amin welches einen Phenylrest als Aromatenrest enthält, hergestellt wurde, hat die folgende idealisierte generische Formel (I):

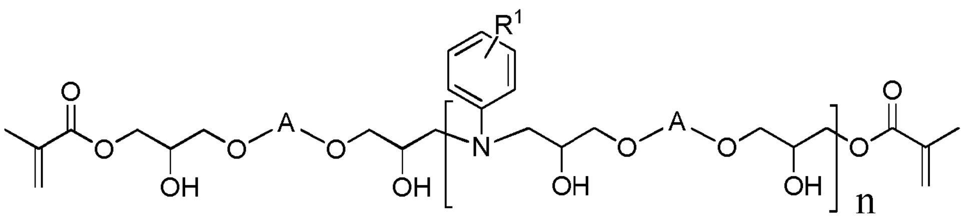

(I).

Die Formel (I) ist idealisiert, da bei der Epoxidöffnung nicht nur sekundäre Alkohole entstehen (typischerweise sind etwa 80% sekundär) und da eine gewisse unregelmäßige Verteilung der Monomere in der Formel möglich ist.

$R^1$ ist in der Formel (I) optional, das heißt, der Phenylrest in Formel (I) kann auch unsubstituiert sein. Wie bereits weiter oben beschrieben, ist bei einem primären aromatischen Amin der Aromatenrest, hier der Phenylrest, bevorzugt jedoch mit einem, zwei oder drei Resten $R^1$ substituiert.

Der Phenylring steht in der gezeigten generischen Formel (I) als Platzhalter für einen Aromatenrest.

In der Formel (I) befindet sich der optional am Aromatenrest vorhandene Substituent $R^1$ in ortho-, meta- oder para-Position zum Stickstoff N, und zwar in derselben Position wie im freien Amin, das zur Herstellung verwendet wurde. Die meta- und die para-Position sind bevorzugt. $R^1$ ist im gebundenen Amin derselbe Substituent wie im freien primären Amin, das zur Herstellung des gebundenen Amins verwendet wurde.

In der gezeigten generischen Formel (I) ist n eine ganze Zahl von 1 bis 20, bevorzugt von 1 bis 10, bevorzugter von 1 bis 7. Besonders bevorzugt ist n eine ganze Zahl von 1 bis 5, ganz besonders bevorzugt von 1 bis 3. Es ist aufgrund der Herstellungsweise für den Amin-Beschleuniger klar, dass der Wert n im aus der erfindungsgemäßen Synthese resultierenden Amin-Beschleuniger (der eine Mischung von Molekülen mit verschiedenen Werten n sein kann) ein Durchschnittswert aus den Einzelwerten für alle Moleküle ist und somit auch eine nicht-ganze Zahl sein kann. Bevorzugt ist der Durchschnittswert von n für den Amin-Beschleuniger ein Wert von etwa 0,9 bis etwa 10, bevorzugter von etwa 1 bis etwa 7, und besonders bevorzugt von etwa 1 bis etwa 5. Ganz besonders bevorzugt ist n ein Wert von etwa 2 bis etwa 3, beispielsweise etwa 2,7.

Der Wert von n wird mittels Gelpermeationschromatographie (GPC; Säule (Polymer Standard Service; Modifiziertes Styrol-Divinylbenzol-Copolymer-Netzwerk): PSS 5$\mu$m SDV 50Å 100Å 1000Ä; Eluent: THF; Kalibrationsstandard: Polystyrol) und der folgenden Formel ermittelt ($M_w$: massemittleres Molekulargewicht; M: Molekulargewicht, berechnet mit n = 1):

$$< n > = \frac{Mw\ (GPC)}{M\ (berechnet\ mit\ n = 1)}$$

Aus prozesstechnischen Gründen sind höhere Molekulargewichte und Molekulargewichtsverteilungen vorteilhaft, was auch Vorteile bei der REACH-Registrierung mit sich bringt.

In der gezeigten generischen Formel (I) bedeutet A den Rest des Diols, welches im dem Diglycidylether enthalten ist. Die Diole, welche erfindungsgemäß verwendet werden, sind weiter oben definiert.

In der gezeigten generischen Formel (II) hat $R^1$ die weiter oben angegebene Bedeutung.

Ein erfindungsgemäßer reaktiver Amin-Beschleuniger, der mit einem sekundären aromatischen Amin welches einen Phenylrest als Aromatenrest enthält, hergestellt wurde, hat die folgende generische Formel (II):

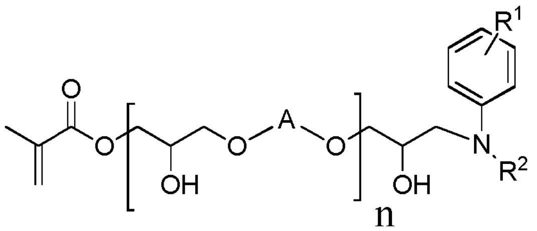

(II).

In der Formel (II) befindet sich der optional am Aromatenrest vorhandene Substituent $R^1$ in ortho-, meta- oder para-Position zum Stickstoff N, und zwar in derselben Position wie im freien Amin, das zur Herstellung verwendet wurde. Die meta- und die para-Position sind bevorzugt. $R^1$ und $R^2$ sind im gebundenen Amin derselbe Substituent wie im freien sekundären Amin, das zur Herstellung des gebundenen Amins verwendet wurde.

In der gezeigten generischen Formel (II) ist n eine ganze Zahl von 1 bis 10, bevorzugt von 1 bis 5, bevorzugter von 1 bis 2. Besonders bevorzugt ist n eine ganze Zahl von 1 bis 5, ganz besonders bevorzugt von 1 bis 2. Es ist aufgrund der Herstellungsweise für den Glycidylether klar, dass der Wert n aus der Synthese des Glycidylethers (der eine Mischung von Molekülen mit verschiedenen Werten n sein kann) ein Durchschnittswert aus den Einzelwerten für alle Moleküle ist und somit auch eine nicht-ganze Zahl sein kann. Bevorzugt ist der Durchschnittswert von n für den Amin-Beschleuniger ein Wert von etwa 0,9 bis etwa 10, bevorzugter von etwa 1 bis etwa 5, und besonders bevorzugt von etwa 1 bis etwa 2. Ganz besonders bevorzugt ist n ein Wert von etwa 1 bis etwa 1,5.

In der gezeigten generischen Formel (II) bedeutet A den Rest des Diols, welches in dem Diglycidylether enthalten ist. Die Diole, welche erfindungsgemäß verwendet werden, sind weiter oben definiert.

In der gezeigten generischen Formel (II) haben $R^1$ und $R^2$ die weiter oben angegebene Bedeutung.

Der Phenylring steht in der gezeigten generischen Formel (II) als Platzhalter für einen Aromatenrest.

Ein erfindungsgemäßer reaktiver Amin-Beschleuniger, der mit Neopentylglycol und einem sekundären aromatischen Amin hergestellt wurde, hat z.B. für n = 1 die folgende Formel:

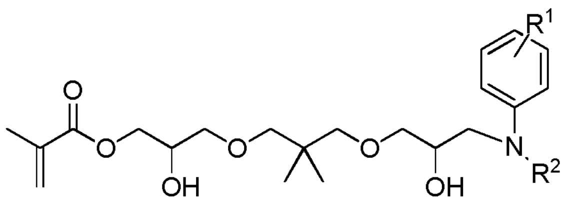

Zum besseren Verständnis der Erfindung werden die folgenden Erläuterungen zur hierin verwendeten Terminologie als sinnvoll erachtet.

Im Sinne der Erfindung bedeuten die verwendeten Begriffe:

- *"Backbone-Harz"* ein üblicherweise festes oder hochviskoses radikalisch polymerisierbares Harz, welches durch Polymerisation (z.B. nach Zugabe eines Initiators in Gegenwart eines Beschleunigers - welcher erfindungsgemäß der reaktive Amin-Beschleuniger ist) härtet;

- *"Reaktivharz-Masterbatch"* das Reaktionsprodukt der Reaktion zur Herstellung des Backbone-Harzes, also eine Mischung aus Backbone-Harz, Inhibitor und weiteren Bestandteilen (z.B. Katalysator) der Reaktionsmischung;

- *"Reaktivharz"* eine Mischung aus Reaktivharz-Masterbatch, einem oder mehreren Inhibitoren, einem Reaktivverdünner und gegebenenfalls weiteren Additiven; das Reaktivharz ist typischerweise flüssig oder viskos und kann zu einer Reaktivharzkomponente weiterverarbeitet werden; hierin wird das Reaktivharz auch als *"Harzmischung"* bezeichnet;

- *"Inhibitor"* einen Stoff, der eine unerwünschte radikalische Polymerisation während der Synthese oder der Lagerung eines Harzes oder einer Harz-haltigen Zusammensetzung unterdrückt (diese Stoffe werden in Fachkreisen auch als *"Stabilisator"* bezeichnet) bzw. der eine radikalische Polymerisation eines Harzes nach Zugabe eines Initiators (üblicherweise in Verbindung mit einem Beschleuniger) zeitlich verzögert (diese Stoffe werden in Fachkreisen auch als *"Inhibitor"* bezeichnet - die jeweilige Bedeutung des Begriffes erschließt sich aus dem Kontext);

- *"Initiator"* einen Stoff, der (üblicherweise in Kombination mit einem Beschleuniger) reaktionsinitiierende Radikale bildet;

- *"Beschleuniger"* ein Reagenz, welches mit dem Initiator eine Reaktion eingeht, so dass bereits bei niedrigen Temperaturen durch den Initiator größere Mengen an Radikalen erzeugt werden, oder welches die Zerfallsreaktion des Initiators katalysiert;

- *"Amin-Beschleuniger",* ein Beschleuniger auf Basis eines Amins, insbesondere eines aromatischen Amins;

- *"reaktiver Amin-Beschleuniger"* ein Amin-Beschleuniger, welcher eine oder zwei $\alpha,\beta$-ungesättigte Carbonsäureestergruppen enthält;

- *"Co-Beschleuniger"* ein Reagenz, welches in die Beschleunigungsreaktion entweder katalytisch oder stöchiometrisch eingreift, um z.B. den Beschleuniger zurückzubilden, die Radikalproduktion pro Zeiteinheit zu moderieren, die Beschleunigungstemperatur noch weiter abzusenken oder eine Kombination dieser oder weiterer Effekte zu bewirken;

- *"Reaktivverdünner"* flüssige oder niedrigviskose Monomere und Backbone-Harze, welche andere Backbone-Harze oder den Reaktivharz-Masterbatch verdünnen und dadurch die zu deren Applikation notwendige Viskosität verleihen, zur Reaktion mit dem Backbone-Harz befähigte funktionelle Gruppen enthalten und bei der Polymerisation (Härtung) zum überwiegenden Teil Bestandteil der gehärteten Masse (z.B. des Mörtels) werden; Reaktivverdünner werden auch co-polymerisierbares Monomer genannt;

- *"Gelzeit",* $t_{g25°C}$, die Zeit (t) der Härtungsphase eines wie hierin definierten Reaktivharzes ($t_{hg25°C}$) oder einer wie hierin definierten Reaktivharzkomponente ($t_{mg25°C}$), in der sich die Temperatur von einer Starttemperatur von 25°C bei einer Gelzeitmessung auf beispielsweise 50°C erhöht; ein Verfahren zur Bestimmung der Gelzeit ist in den Beispielen beschrieben;

- *"maximale Reaktivitätstemperatur $T_{max}$"* diejenige Temperatur, bei welcher der Temperaturverlauf bei einer Reaktivitätsmessung (z.B. der in den Beispielen beschriebenen Gelzeitmessung) ein Maximum durchläuft;

- *"Abschluss der Reaktion"* bzw. *"Reaktionsende"* oder *"Reaktionsabschluss"* den Zeitpunkt, zu dem eine Reaktion vollständig abgelaufen ist; erkennbar ist dies bei einer chemischen Reaktion wie z.B. der Reaktion zur Herstellung des Backbone-Harzes in der Regel daran, dass die mit der Reaktion verbundene Exothermie endet;

- *"Reaktivharzkomponente"* eine flüssige oder viskose Mischung aus Reaktivharz und Füllstoffen und optional weiteren Komponenten, z.B. Additiven; typischerweise ist die Reaktivharzkomponente eine der beiden Komponenten eines Zweikomponenten-Reaktivharz-Systems zur chemischen Befestigung;

- *"Härterkomponente"* eine Zusammensetzung, die einen Initiator für die Polymerisation eines Backbone-Harzes enthält; die Härterkomponente kann fest oder flüssig sein und neben dem Initiator ein Lösungsmittel sowie Füllstoffe und/oder Additive enthalten; typischerweise ist die Härterkomponente neben der Reaktivharzkomponente die andere der beiden Komponenten eines Zweikomponenten-Reaktivharz-Systems zur chemischen Befestigung;

- *"Zweikomponenten-System"* bzw. *"Zweikomponenten-Reaktivharz-System"* ein Reaktivharz-System, das zwei voneinander getrennt gelagerte Komponenten, eine Reaktivharzkomponente (A) und eine Härterkomponente (B), umfasst, so dass eine Härtung des in der Reaktivharzkomponente enthaltenen Backbone-Harzes erst nach dem Mischen der beiden Komponenten erfolgt;

- *"Mehrkomponenten-System"* bzw. *"Mehrkomponenten-Reaktivharz-System"* ein Reaktivharz-System, das mehrere voneinander getrennt gelagerte Komponenten umfasst, unter anderem eine Reaktivharzkomponente (A) und eine Härterkomponente (B), so dass eine Härtung des in der Reaktivharzkomponente enthaltenen Backbone-Harzes erst nach dem Mischen aller Komponenten erfolgt;

- *"(Meth)acryl.../..(meth)acryl...",* dass sowohl die "Methacryl.../...methacryl..."- als auch die "Acryl.../...acryl..."-Verbindungen gemeint sein sollen; bevorzugt sind in der vorliegenden Erfindung "Methacryl.../...methacryl..."-Verbindungen gemeint;

- *"Epoxy(meth)acrylat"* ein Epoxidharz, das Acrylat- oder Methacrylatgruppen aufweist und im wesentlichen Epoxygruppen-frei ist;

- *"Alkyl"* einen gesättigten Kohlenwasserstoffrest, der verzweigt oder unverzweigt sein kann; bevorzugt ein $C_1$-$C_{20}$-Alkyl, besonders bevorzugt ein $C_1$-$C_4$-Alkyl, also ein Alkyl ausgewählt aus der Gruppe bestehend aus Methyl, Ethyl, n-Propyl, iso-Propyl, n-Butyl, iso-Butyl, und tert-Butyl; Methyl, Ethyl und tert-Butyl sind besonders bevorzugt, und ganz besonders bevorzugt ist Methyl;

- "Hydroxyalkyl" ein Alkyl, das mindestens eine Hydroxylgruppe als Substituenten trägt; bevorzugt eine Hydroxylgruppe;

- "Alkenyl" einen ungesättigten Kohlenwasserstoffrest mit mindestens einer und maximal fünf Doppelbindungen, bevorzugt eine, der verzweigt oder unverzweigt sein kann; bevorzugt ein $C_2$-$C_{20}$-Alkenyl, besonders bevorzugt ein $C_2$-$C_6$-Alkenyl, also ein Alkenyl ausgewählt aus der Gruppe bestehend aus Ethenyl, Propenyl, Butenyl, Pentenyl und Hexenyl; Ethenyl, Propenyl und Butenyl sind besonders bevorzugt, und ganz besonders bevorzugt ist Ethenyl;

- "Hydroxy-Alkenyl" ein Alkenyl, das mindestens eine Hydroxylgruppe als Substituenten trägt, bevorzugt eine Hydroxylgruppe;

- "Alkinyl" einen ungesättigten Kohlenwasserstoffrest mit mindestens einer und maximal fünf Dreifachbindungen, bevorzugt eine, der verzweigt oder unverzweigt sein kann; bevorzugt ein $C_2$-$C_{20}$-Alkinyl, besonders bevorzugt ein $C_2$-$C_6$-Alkinyl, also ein Alkinyl ausgewählt aus der Gruppe bestehend aus Ethinyl, Propinyl, Butinyl, Pentinyl und Hexinyl; Ethinyl, Propinyl und Butinyl sind besonders bevorzugt, und ganz besonders bevorzugt ist Ethinyl;

- "Hydroxy-Alkinyl" ein Alkinyl, das mindestens eine Hydroxylgruppe als Substituenten trägt; bevorzugt eine Hydroxylgruppe;

- *"kalthärtend"* dass eine Harzmischung oder ein Reaktivharz-System bei Raumtemperatur vollständig aushärten kann;

- *"ein", "eine", "einer"* als Artikel vor einer chemischen Verbindungsklasse, z.B. vor dem Wort "Epoxymethacrylat", dass eine oder mehrere unter diese chemische Verbindungsklasse fallende Verbindungen, z.B. verschiedene Epoxymethacrylate, gemeint sein können. In einer bevorzugten Ausführungsform ist mit diesem Artikel nur eine einzelne Verbindung gemeint;

- *"mindestens ein", "mindestens eine", "mindestens einer"* zahlenmäßig *"ein oder mehrere".* In einer bevorzugten Ausführungsform ist mit diesem Begriff zahlenmäßig *"ein", "eine", "einer"* gemeint;

- *"enthalten", "umfassen"* und *"beinhalten",* dass neben den genannten Bestandteilen noch weitere vorhanden sein können. Diese Begriffe sind einschließlich gemeint und umfassen daher auch *"bestehen aus". "Bestehen aus"* ist abschließend gemeint und bedeutet, dass keine weiteren Bestandteile vorhanden sein können. In einer bevorzugten Ausführungsform bedeuten die Begriffe *"enthalten", "umfassen"* und *"beinhalten"* den Begriff *"bestehen aus";*

- *"etwa"* oder *"circa"* oder *"ca."* vor einem Zahlenwert einen Bereich von ± 5% dieses Wertes, bevorzugt ± 2% dieses Wertes, bevorzugter ± 1% dieses Wertes, besonders bevorzugt ± 0% dieses Wertes (also genau diesen Wert);

- ein durch Zahlen begrenzter Bereich, z.B. *"von 80°C bis 120°C",* dass die beiden Eckwerte und jeder Wert innerhalb

dieses Bereichs einzeln offenbart sind.

Alle in diesem Text genannten Normen (z.B. DIN-Normen) wurden in der zum Anmeldetag dieser Anmeldung aktuellen Ausgabe verwendet.

Die Herstellung des erfindungsgemäßen reaktiven Amin-Beschleunigers erfolgt wie oben beschrieben, indem (1) ein aromatisches primäres oder sekundäres Amin umgesetzt wird mit (2) einem Diglycidylether und (3) einer $\alpha,\beta$-ungesättigten Carbonsäure. Die Reaktion erfolgt typischerweise in Gegenwart von (4) einem Katalysator. Optional kann (5) ein Inhibitor im Reaktionsgemisch vorhanden sein. Die Ausgangsverbindungen werden vermischt und miteinander zur Reaktion gebracht. Typischerweise werden alle Herstellungsschritte unter Rühren ausgeführt, es sind jedoch auch andere Durchmischungsarten denkbar. Nach Beendigung der Reaktion zur Herstellung des reaktiven Amin-Beschleunigers werden zur anschließenden Herstellung des Reaktivharzes weitere Komponenten, insbesondere das Backbone-Harz zugesetzt.

Alternativ können einzelne Komponenten des Reaktivharzes, insbesondere thermisch stabile und nicht reaktive Komponenten, der Reaktionsmischung zur Herstellung des reaktiven Amin-Beschleunigers bereits vor Beginn der Reaktion zum reaktiven Amin-Beschleuniger zugesetzt werden. Der Zusatz dieser weiteren Komponenten nach Abschluss der Reaktion zum reaktiven Amin-Beschleuniger ist jedoch bevorzugt.

Ein erfindungsgemäßer reaktiver Amin-Beschleuniger ist bevorzugt eine Verbindung der Formel (I) oder (II), wie oben dargestellt. Wenn sowohl primäres wie auch sekundäres aromatisches Amin zur Herstellung des erfindungsgemäßen reaktiven Amin-Beschleunigers verwendet wurden, enthält der reaktive Amin-Beschleuniger erfindungsgemäß sowohl Verbindungen der Formel (I) als auch der Formel (II).

Ein erfindungsgemäßes Reaktivharz enthält mindestens einen erfindungsgemäßen reaktiven Amin-Beschleuniger, mindestens ein Backbone-Harz, mindestens einen Reaktivverdünner und mindestens einen Inhibitor. Da der reaktive Amin-Beschleuniger und das Backbone-Harz nach ihrer Herstellung typischerweise ohne Isolierung für die Herstellung des Reaktivharzes verwendet werden, sind in der Regel auch noch die neben dem reaktiven Amin-Beschleuniger in dessen Reaktionsmischung und die neben dem Backbone-Harz im Reaktivharz-Masterbatch enthaltenen weiteren Bestandteile im erfindungsgemäßen Reaktivharz vorhanden.

In einem bevorzugten Gegenstand der Erfindung enthält das erfindungsgemäße Reaktivharz eine Mischung aus zwei oder mehr, bevorzugt aus zwei erfindungsgemäßen reaktiven Amin-Beschleunigern.

Die in den Beispielen beschriebenen Beschleuniger-Kombinationen charakterisieren eine bevorzugte Ausführungsform der Erfindung.

Als Backbone-Harze sind erfindungsgemäß ethylenisch ungesättigte Verbindungen, Verbindungen mit Kohlenstoff-Kohlenstoff-Dreifachbindungen und Thiol-Yne/Ene-Harze geeignet, wie sie dem Fachmann bekannt sind.

Von diesen Verbindungen ist die Gruppe der ethylenisch ungesättigten Verbindungen bevorzugt, die Styrol und Derivate davon, (Meth)acrylate, Vinylester, ungesättigte Polyester, Vinylether, Allylether, Itaconate, Dicyclopentadien-Verbindungen und ungesättigte Fette umfasst, wovon insbesondere ungesättigte Polyesterharze und Vinylesterharze geeignet und beispielsweise in den Anmeldungen EP 1 935 860 A1, DE 195 31 649 A1 und WO 10/108939 A1 beschrieben sind. Vinylesterharze sind dabei aufgrund ihrer hydrolytischen Beständigkeit und ausgezeichneten mechanischen Eigenschaften am stärksten bevorzugt.

Beispiele geeigneter ungesättigter Polyester, die in der erfindungsgemäßen Harzmischung verwendet werden können, werden in folgende Kategorien eingeteilt, wie sie durch M. Malik et al. in J. M. S. - Rev. Macromol. Chem. Phys., C40(2 and 3), p.139-165 (2000) klassifiziert wurden:

(1) Ortho-Harze: diese basieren auf Phthalsäureanhydrid, Maleinsäureanhydrid oder Fumarsäure und Glykolen, wie 1,2-Propylenglykol, Ethylenglykol, Diethylenglykol, Triethylenglykol, 1,3-Propylenglykol, Dipropylenglykol, Tripropylenglykol, Neopentylglykol oder hydrogeniertes Bisphenol-A;

(2) Iso-Harze: diese werden aus Isophthalsäure, Maleinsäureanhydrid oder Fumarsäure und Glykolen hergestellt. Diese Harze können höhere Anteile an Reaktivverdünnern enthalten als die Ortho-Harze;

(3) Bisphenol-A-fumarate: diese basieren auf ethoxyliertem Bisphenol-A und Fumarsäure;

(4) HET-Säure-Harze (Hexachloro-endo-methylen-tetrahydrophthalsäure-Harze): sind Harze, die aus Chlor/Brom enthaltenden Anhydriden oder Phenolen bei der Herstellung von ungesättigten Polyesterharzen gewonnen werden.

Neben diesen Harzklassen können noch die sogenannten Dicyclopentadien-Harze (DCPD-Harze) als ungesättigte Polyesterharze unterschieden werden. Die Klasse der DCPD-Harze wird entweder durch Modifikation eines der oben genannten Harztypen durch Diels-Alder-Reaktion mit Cyclopentadien erhalten, oder sie werden alternativ durch eine erste Reaktion eines Diacids, z.B. Maleinsäure, mit Dicyclopentadien, und anschließend durch eine zweite Reaktion,

der gewöhnlichen Herstellung eines ungesättigten Polyesterharzes erhalten, wobei man bei letzterem von einem DCPD-Maleatharz spricht.

Das ungesättigte Polyesterharz hat bevorzugt ein Molekulargewicht Mn im Bereich von 500 bis 10.000 Dalton, stärker bevorzugt im Bereich von 500 bis 5000 und noch stärker bevorzugt im Bereich von 750 bis 4000 (nach ISO 13885-1). Das ungesättigte Polyesterharz hat einen Säurewert im Bereich 0 bis 80 mg KOH/g Harz, bevorzugt im Bereich von 5 bis 70 mg KOH/g Harz (nach ISO 2114-2000). Wird ein DCPD-Harz als ungesättigtes Polyesterharz verwendet beträgt der Säurewert bevorzugt 0 bis 50 mg KOH/g Harz.

Im Sinne der Erfindung sind Vinylesterharze Oligomere oder Polymere mit mindestens einer (Meth)acrylat-Endgruppe, so genannte (Meth)acrylat-funktionalisierte Harze, wozu auch Urethan(meth)acrylat-Harze und Epoxy(meth)acrylate zählen, welche besonders bevorzugt sind.

Vinylesterharze, die nur in Endstellung ungesättigten Gruppen aufweisen, werden zum Beispiel durch Umsetzung von Epoxid-Oligomeren oder -Polymeren (z.B. Bisphenol A-digylcidylether, Epoxide vom Phenol-Novolak-Typ oder Epoxid-Oligomere auf der Basis von Tetrabrombisphenol A) mit beispielsweise (Meth)acrylsäure oder (Meth)acrylamid erhalten. Bevorzugte Vinylester-Harze sind (Meth)acrylat-funktionalisierte Harze und Harze, die durch Umsetzung eines Epoxid-Oligomeren oder -Polymeren mit Methacrylsäure oder Methacrylamid, bevorzugt mit Methacrylsäure, erhalten werden. Beispiele solcher Verbindungen sind aus den Anmeldungen US 3297745 A, US 3772404 A, US 4618658 A, GB 2217722 A1, DE 3744390 A1 und DE 4131457 A1 bekannt.

In diesem Zusammenhang wird auf die Anmeldung US 2011071234 A1 verwiesen.

Das Vinylester-Harz hat bevorzugt ein Molekulargewicht Mn im Bereich von 500 bis 3000 Dalton, stärker bevorzugt 500 bis 1500 Dalton (nach ISO 13885-1). Das Vinylester-Harz hat einen Säurewert im Bereich von 0 bis 50 mg KOH/g Harz, bevorzugt im Bereich von 0 bis 30 mg KOH/g Harz (nach ISO 2114-2000).

Als Vinylesterharz besonders geeignet sind ethoxyliertes Bisphenol-A-di(meth)acrylat mit einem Ethoxylierungsgrad von 2 bis 10, vorzugsweise von 2 bis 4, difunktionelle, trifunktionelle oder höherfunktionelle Urethan(meth)acrylat-Oligomere oder Mischungen dieser härtbaren Bestandteile.

Ganz besonders geeignet sind die bekannten Reaktionsprodukte aus Di- oder Polyisocyanaten und Hydroxyalkylmethylacrylaten, wie sie beispielsweise in der DE 2 312 559 A1 beschrieben sind, Addukte aus (Di)Isocyanaten und 2,2-Propanbis-[3-(4-phenoxy)-1,2-hydroxypropan-1-methacrylat] nach der US-PS 3 629 187 sowie die Addukte aus Isocyanaten und Methacryloylalkylethern, -alkoxybenzolen bzw. -alkoxycycloalkanen, wie sie in der EP 44352 A1 beschrieben sind. In diesem Zusammenhang wird auf die DE 2312559 A1, DE 19902685 A1, EP 0684906 A1, DE 4111828 A1 und DE 19961342 A1 verwiesen. Selbstverständlich können auch Gemische aus geeigneten Monomeren verwendet werden.

All diese Harze, die erfindungsgemäß verwendet werden können, können gemäß dem Fachmann bekannten Verfahren modifiziert werden, um zum Beispiel geringere Säurezahlen, Hydroxidzahlen oder Anhydridzahlen zu erreichen, oder durch das Einbringen von flexiblen Einheiten in das Grundgerüst flexibler gemacht werden, und dergleichen.

Darüber hinaus kann das Harz noch andere reaktive Gruppen, die mit einem Radikalinitiator, wie Peroxiden, polymerisiert werden können, enthalten, zum Beispiel reaktive Gruppen, die von der Itaconsäure, Citraconsäure und allylischen Gruppen und dergleichen abgeleitet sind, wie sie beispielsweise in der WO 2010/108939 A1 (Itaconsäureester) beschrieben sind.

Der prozentuale Anteil (in Gew.-% des Reaktivharzes) von Backbone-Harz in dem erfindungsgemäßen Reaktivharz beträgt vorteilhafterweise mehr als etwa 5%, bevorzugt mehr als etwa 15%, und besonders bevorzugt mehr als etwa 20%. Der prozentuale Anteil (in Gew.-% des Reaktivharzes) von Backbone-Harz in dem Reaktivharz beträgt vorteilhafterweise von etwa 5% bis etwa 90%, bevorzugt von etwa 8% bis etwa 80%, bevorzugter von etwa 10% bis etwa 60%, bevorzugter von etwa 20% bis etwa 55%, noch bevorzugter von etwa 25% bis etwa 55%, besonders bevorzugt von etwa 25% bis etwa 50% und ganz besonders bevorzugt von etwa 28% bis etwa 45%.

Der Anteil (in mmol Amin pro 100 g des Reaktivharzes) von erfindungsgemäßem reaktiven Amin-Beschleuniger im erfindungsgemäßen Reaktivharz beträgt von 0,5 bis 50, bevorzugt von 1 bis 20, besonders bevorzugt von 5 bis 15 mmol Amin/100g Reaktivharz. Wird ein Gemisch aus mehreren erfindungsgemäßen reaktiven Amin-Beschleunigern im erfindungsgemäßen Reaktivharz verwendet, so bezieht sich der Anteil auf das Gemisch.

Sowohl zur Stabilisierung des Reaktivharzes oder der das Reaktivharz enthaltenden Reaktivharzkomponente (A) oder anderer das Reaktivharz enthaltender Zusammensetzungen als auch zur Einstellung der Harzreaktivität sind im erfindungsgemäßen Reaktivharz ein oder mehrere Inhibitoren vorhanden.

Hierfür sind die für radikalisch polymerisierbare Verbindungen üblicherweise verwendeten Inhibitoren geeignet, wie sie dem Fachmann bekannt sind. Bevorzugt sind diese Inhibitoren unter phenolischen Inhibitoren und nicht-phenolischen Inhibitoren, insbesondere Phenothiazinen, ausgewählt.

Als phenolische Inhibitoren sind Phenole, wie 2-Methoxyphenol, 4-Methoxyphenol, 2,6-Di-tert-butyl-4-methylphenol, 2,4-Di-tert-butylphenol, 2,6-Di-tert-butylphenol, 2,4,6-Trimethylphenol, 2,4,6-Tris(dimethylaminomethyl)phenol, 4,4'-Thio-bis(3-methyl-6-tert-butylphenol), 4,4'-Isopropylidendiphenol, 6,6'-Di-tert-butyl-4,4'-bis(2,6-di-tert-butylphenol), 1,3,5-Trimethyl-2,4,6-tris(3,5-di-tert-butyl-4-hydroxybenzyl)benzol, 2,2'-Methylen-di-p-cresol, Catechole, wie Brenzkatechin, und Catecholderivate, wie Butylbrenzkatechine, wie 4-tert-Butylbrenzkatechin und 4,6-Di-tert-butylbrenzkatechin, Hydrochinone, wie Hydrochinon, 2-Methylhydrochinon, 2-tert-Butylhydrochinon, 2,5-Di-tert-butylhydrochinon, 2,6-Di-tert-butylhydrochinon, 2,6-Dimethylhydrochinon, 2,3,5-Trimethylhydrochinon, Benzochinon, 2,3,5,6-Tetrachloro-1,4-benzochinon, Methylbenzochinon, 2,6-Dimethylbenzochinon, Naphthochinon, oder Gemische von zweien oder mehreren davon, geeignet. Diese Inhibitoren sind oft Bestandteil von kommerziellen radikalisch härtenden Reaktivharzkomponenten.

Als nicht-phenolische Inhibitoren kommen vorzugsweise Phenothiazine, wie Phenothiazin und/oder Derivate oder Kombinationen davon, oder stabile organische Radikale, wie etwa Galvinoxyl- und *N*-Oxyl-Radikale, insbesondere vom Piperidinyl-*N*-oxyl- oder Tetrahydropyrrol-*N*-oxyl-Typ, in Betracht, wie Aluminium-*N*-nitrosophenylhydroxylamin, Diethylhydroxylamin, Oxime, wie Acetaldoxim, Acetonoxim, Methylethylketoxim, Salicyloxim, Benzoxim, Glyoxime, Dimethylglyoxim, Aceton-O-(benzyloxycarbonyl)oxim, TEMPOL, TEMPO und dergleichen.

Ferner können in para-Stellung zur Hydroxylgruppe substituierte Pyrimidinol- oder Pyridinol-Verbindungen, wie sie in der Patentschrift DE 10 2011 077 248 B1 beschrieben sind, als Inhibitoren eingesetzt werden.

Als stabile *N*-Oxyl-Radikale können beispielsweise solche verwendet werden, wie sie in der DE 199 56 509 A1 und der DE 195 31 649 A1 beschrieben sind. Derartige stabile Nitroxylradikale sind vom Typ Piperidinyl-*N*-oxyl oder Tetrahydropyrrol-*N*-oxyl oder eine Mischung daraus.

Bevorzugte stabile Nitroxylradikale sind ausgewählt aus der Gruppe bestehend aus 1-Oxyl-2,2,6,6-tetramethylpiperidin, 1-Oxyl-2,2,6,6-tetramethylpiperidin-4-ol (ebenso als TEMPOL bezeichnet), 1-Oxyl-2,2,6,6-tetramethylpiperidin-4-on (ebenso als TEMPON bezeichnet), 1-Oxyl-2,2,6,6-tetramethyl-4-carboxyl-piperidin (ebenso als 4-Carboxy-TEMPO bezeichnet), 1-Oxyl-2,2,5,5-tetramethylpyrrolidin, 1-Oxyl-2,2,5,5-tetramethyl-3-carboxylpyrrolidin (ebenso als 3-Carboxy-PROXYL bezeichnet) und Mischungen aus zwei oder mehreren dieser Verbindungen, wobei 1-Oxyl-2,2,6,6-tetramethylpiperidin-4-ol (TEMPOL) besonders bevorzugt ist.

Bevorzugt sind der oder die Inhibitoren ausgewählt aus der Gruppe bestehend aus *N*-Oxyl-Radikalen, Catecholen, Catecholderivaten und Phenothiazinen und ein Gemisch von zwei ider mehreren davon. Besonders bevorzugt sind der oder die Inhibitoren ausgewählt aus der Gruppe bestehend aus Tempol, Catecholen und Phenothiazinen. Ganz besonders bevorzugt sind die in den Beispielen verwendeten Inhibitoren, bevorzugt etwa in den in den Beispielen angegebenen Mengen.

Die Inhibitoren können, abhängig von den gewünschten Eigenschaften des Reaktivharzes, entweder alleine oder als Kombination von zweien oder mehreren davon verwendet werden. Die Kombination von phenolischen und nicht-phenolischen Inhibitoren ist bevorzugt.

Der Inhibitor oder die Inhibitormischung wird zugesetzt in der Fachwelt bekannten üblichen Mengen, bevorzugt in einer Menge von etwa 0,0005 bis etwa 2 Gew.-% (bezogen auf das - letztendlich damit hergestellte - Reaktivharz), stärker bevorzugt von etwa 0,01 bis etwa 1 Gew.-% (bezogen auf das Reaktivharz), noch stärker bevorzugt von etwa 0,05 bis etwa 1 Gew.-% (bezogen auf das Reaktivharz).

Das erfindungsgemäße Reaktivharz enthält mindestens einen Reaktivverdünner.

Geeignete Reaktivverdünner sind niederviskose, radikalisch co-polymerisierbare Verbindungen, bevorzugt kennzeichnungsfreie Verbindungen.

Geeignete Reaktivverdünner sind in den Anmeldungen EP 1 935 860 A1 und DE 195 31 649 A1 beschrieben. Vorzugsweise enthält das Reaktivharz als Reaktivverdünner einen (Meth)acrylsäureester, wobei besonders bevorzugt aliphatische oder aromatische $C_5$-$C_{15}$-(Meth)acrylate ausgewählt werden. Geeignete Beispiele umfassen: 2-, 3-Hydroxypropyl(meth)acrylat (HP(M)A), 1,2-Ethandioldi(meth)acrylat, 1,3-Propandioldi(meth)acrylat, 1,3-Butandioldi(meth)acrylat, 1,4-Butandioldi(meth)acrylat, Trimethylolpropantri(meth)acrylat, Phenethyl(meth)acrylat, Tetrahydrofurfuryl(meth)acrylat, Ethyltriglykol(meth)acrylat, *N,N*-Dimethylaminoethyl(meth)acrylat, *N,N*-Dimethylaminomethyl(meth)acrylat, Acetoacetoxyethyl(meth)acrylat, Isobornyl(meth)acrylat, 2-Ethylhexyl(meth)acrylat, Diethylenglykoldi(meth)acrylat, Methoxypolyethylenglykolmono(meth)acrylat, Trimethylcyclohexyl(meth)acrylat, 2-Hydroxyethyl(meth)acrylat, Dicyclopentenyloxyethyl(meth)acrylat und/oder Tricyclopentadienyldi(meth)acrylat, Bisphenol-A-(meth)acrylat, Novolakepoxidi(meth)acrylat, Di-[(meth)acryloyl-maleoyl]-tricyclo-5.2.1.0.$^{2.6}$-decan, Dicyclopentenyloxyethylcrotonat, 3-(Meth)acryloyl-oxymethyl-tricylo-5.2.1.0.$^{2.6}$-decan, 3-(Meth)cyclopentadienyl(meth)acrylat, und Decalyl-2-(meth)acrylat; PEG-di(meth)acrylate, wie PEG200-di(meth)acrylat, Tetraehtylenglykoldi(meth)acrylat, Solketal(meth)acrylat, Cyclohexyl(meth)acrylat, Phenoxyethyldi(meth)acrylat, Methoxyethyl(meth)acrylat, *tert*-Butyl(meth)acrylat und Norbornyl(meth)acrylat. Methacrylate sind gegenüber Acrylaten bevorzugt. Besonders bevorzugt sind 2- und 3-Hydroxypropylmethacrylat (HPMA), 1,2-Ethandioldimethacrylat, 1,4-Butandioldimethacrylat (BDDMA), 1,3-Butandioldimethacrylat, Trimethylolpropantrimethacrylat, Acetoacetoxyethylmethacrylat, Isobornylmethacrylat, Bisphenol-A-methacrylat, Trimethylcyclohexylmethacrylat, 2-Hydroxyethylmethacrylat, PEG200-dimethacrylat und Norbornylmethacrylat. Ganz besonders bevorzugt sind 1,4-Butandioldimethacrylat und ein Gemisch aus 2- und 3-Hydroxypropylmethacrylat (HPMA), oder eine Mischung aus diesen drei Methacrylaten. Am bevorzugtesten ist ein Gemisch aus 2- und 3-Hydroxypropylmethacrylat (HPMA). Grundsätzlich können auch andere übliche radikalisch polymerisierbare Verbindungen, allein oder im Gemisch mit den (Meth)acrylsäureestern, als Reaktivverdünner eingesetzt werden, z. B. Styrol,

α-Methylstyrol, alkylierte Styrole, wie tert-Butylstyrol, Divinylbenzol und Vinyl- sowie Allylverbindungen, wobei die nicht kennzeichnungspflichtigen Vertreter davon bevorzugt sind. Beispiele für derartige Vinyl- oder Allylverbindungen sind Hydroxybutylvinylether, Ethylenglycoldivinylether, 1,4-Butandioldivinylether, Trimethylolpropandivinylether, Trimethylolpropantrivinylether, Mono-, Di-, Tri-, Tetra- und Polyalkylenglycolvinylether, Mono-, Di-, Tri-, Tetra- und Polyalkylenglycolallylether, Adipinsäuredivinylester, Trimethylolpropandiallylether und Trimethylolpropantriallylether.

Bevorzugte Reaktivverdünner sind die in den Beispielen verwendeten Reaktivverdünner, bevorzugt etwa in den in den Beispielen angegebenen Mengen.

Der bzw. die Reaktivverdünner wird bzw. sind bevorzugt in einer Menge von 0 bis etwa 80 Gew.-%, besonders bevorzugt von etwa 10 bis etwa 60 Gew.-%, noch bevorzugter von etwa 20 bis etwa 50 Gew.-%, bezogen auf das Reaktivharz, im erfindungsgemäßen Reaktivharz vorhanden.

Zweckmäßig wird die Härtung des Reaktivharzes mit einem Peroxid als Initiator initiiert. Alle dem Fachmann bekannten Peroxide, die zum Härten von Epoxid(meth)acrylatharzen verwendet werden, können eingesetzt werden. Derartige Peroxide umfassen organische und anorganische Peroxide, entweder flüssig oder fest, wobei auch Wasserstoffperoxid verwendet werden kann. Beispiele geeigneter Peroxide sind Peroxycarbonate (der Formel -OC(O)OO-), Peroxyester (der Formel -C(O)OO-), Diacylperoxide (der Formel -C(O)OOC(O)-), Dialkylperoxide (der Formel -OO-), Hydroperoxide (der Formel -OOH) und dergleichen. Diese können als Oligomer oder Polymer vorliegen. Eine umfassende Reihe an Beispielen für geeignete Peroxide ist zum Beispiel in der Anmeldung US 2002/0091214 A1, Absatz [0018], beschrieben.

Bevorzugt sind die Peroxide aus der Gruppe der organischen Peroxide ausgewählt. Geeignete organische Peroxide sind: tertiäre Alkylhydroperoxide, wie tert-Butylhydroperoxid, und andere Hydroperoxide, wie Cumenhydroperoxid, Peroxyester oder Persäuren, wie tert-Butylperester (z.B. tert-Butylperoxybenzoat), Benzoylperoxid, Peracetate und Perbenzoate, Lauroylperoxid, einschließlich (Di)peroxyester, Perether, wie Peroxydiethylether, Perketone, wie Methylethylketonperoxid. Die als Härter verwendeten organischen Peroxide sind oft tertiäre Perester oder tertiäre Hydroperoxide, d.h. Peroxid-Verbindungen mit tertiären Kohlenstoffatomen, die direkt an eine -O-O-acyl- oder -OOH-Gruppe gebunden sind. Aber auch Gemische dieser Peroxide mit anderen Peroxiden können erfindungsgemäß eingesetzt werden. Die Peroxide können auch gemischte Peroxide sein, d.h. Peroxide, die zwei verschiedene Peroxid-tragende Einheiten in einem Molekül aufweisen. In einer bevorzugten Ausführungsform wird zum Härten Benzoylperoxid (BPO) oder tert-Butylperoxybenzoat verwendet.

Das Peroxid kann in seiner Reinform oder als Bestandteil einer Mischung verwendet werden. Typischerweise wird es als Bestandteil einer Mischung verwendet, insbesondere als Bestandteil einer Härterkomponente (B) eines Reaktivharz-Systems. Die in den Beispielen verwendete Härterkomponente bzw. eine Härterkomponenten mit denselben Bestandteilen ist besonders bevorzugt.

Gegenstand der vorliegenden Erfindung ist auch ein Reaktivharz-System, bestehend aus einer Reaktivharzkomponente (A) und einer Härterkomponente (B). Die Reaktivharzkomponente (A) alleine ist ebenfalls Gegenstand der vorliegenden Erfindung. Besagte Reaktivharzkomponente (A) enthält das erfindungsgemäße Reaktivharz.

Die erfindungsgemäße Reaktivharzkomponente (A) kann neben dem erfindungsgemäßen Reaktivharz Füllstoffe und/oder Additive enthalten. Es sei darauf hingewiesen, dass einige Stoffe sowohl als Füllstoff und, ggf. in modifizierter Form, auch als Additiv verwendet werden können. Beispielsweise dient pyrogene Kieselsäure in ihrer polaren, nicht nachbehandelten Form eher als Füllstoff und in ihrer apolaren, nachbehandelten Form eher als Additiv. In Fällen, in denen genau derselbe Stoff als Füllstoff oder Additiv verwendet werden kann, soll seine Gesamtmenge die hierin festgelegte Obergrenze für Füllstoffe vorteilhafterweise nicht überschreiten.

Zur Herstellung einer Reaktivharzkomponente für bauliche Anwendungen, inbesondere für die chemische Befestigung, können dem erfindungsgemäßen Reaktivharz übliche Füllstoffe zugegeben werden. Diese Füllstoffe sind typischerweise anorganische Füllstoffe, wie beispielsweise weiter unten beschrieben.

Der Anteil des erfindungsgemäßen Reaktivharzes in der Reaktivharzkomponente beträgt bevorzugt von etwa 10 bis etwa 70 Gew.-%, stärker bevorzugt von etwa 30 bis etwa 60 Gew.-%, noch stärker bevorzugt von etwa 35 bis etwa 50 Gew.-%, bezogen auf die Reaktivharzkomponente. Dementsprechend beträgt der Anteil der gesamten Füllstoffe und Additive bevorzugt von etwa 90 bis etwa 30 Gew.-%, stärker bevorzugt von etwa 70 bis etwa 40 Gew.-%, noch stärker bevorzugt von etwa 75 bis etwa 50 Gew.-%, bezogen auf die Reaktivharzkomponente.

Als Füllstoffe finden übliche Füllstoffe, vorzugsweise mineralische oder mineralähnliche Füllstoffe, wie Quarz, Glas, Sand, Quarzsand, Quarzmehl, Porzellan, Korund, Keramik, Talkum, Kieselsäure (z.B. pyrogene Kieselsäure, insbesondere polare, nicht nachbehandelte pyrogene Kieselsäure), Silikate, Aluminiumoxide (z.B. Tonerde), Ton, Titandioxid, Kreide, Schwerspat, Feldspat, Basalt, Aluminiumhydroxid, Granit oder Sandstein, polymere Füllstoffe, wie Duroplaste, hydraulisch härtbare Füllstoffe, wie Gips, Branntkalk oder Zement (z.B. Aluminatzement (oft auch als Tonerdezement bezeichnet) oder Portlandzement), Metalle, wie Aluminium, Ruß, ferner Holz, mineralische oder organische Fasern, oder dergleichen, oder Gemische von zwei oder mehr davon Verwendung. Die Füllstoffe können in beliebigen Formen vorliegen, beispielsweise als Pulver oder Mehl, oder als Formkörper, z.B. in Zylinder-, Ring-, Kugel-, Plättchen-, Stäbchen-, Sattel- oder Kristallform, oder ferner in Faserform (fibrilläre Füllstoffe), und die entsprechenden Grundteilchen

haben vorzugsweise einen maximalen Durchmesser von etwa 10 mm und einen Minimaldurchmesser von etwa 1 nm. Das heißt, der Durchmesser ist etwa 10 mm oder irgendein Wert von weniger als etwa 10 mm, aber mehr als etwa 1 nm. Bevorzugt ist der maximale Durchmesser ein Durchmesser von etwa 5 mm, bevorzugter von etwa 3 mm, noch bevorzugter von etwa 0,7 mm. Ganz besonders bevorzugt ist ein maximaler Durchmesser von etwa 0,5 mm. Der bevorzugtere Minimaldurchmesser ist etwa 10 nm, noch bevorzugter etwa 50 nm, ganz besonders bevorzugt etwa 100 nm. Durchmesserbereiche, die sich durch Kombination dieser maximalen Durchmesser und Minimaldurchmesser ergeben, sind besonders bevorzugt. Bevorzugt und deutlicher verstärkend wirken sich jedoch die globulären, inerten Stoffe (Kugelform) aus. Auch Core-Shell-Partikel, bevorzugt in Kugelform, können als Füllstoffe verwendet werden.

Bevorzugte Füllstoffe sind ausgewählt aus der Gruppe bestehend aus Zement, Kieselsäure, Quarz, Quarzsand, Quarzmehl, und Mischungen aus zwei oder mehreren davon. Für die Reaktivharzkomponente (A) besonders bevorzugt sind Füllstoffe ausgewählt aus der Gruppe bestehend aus Zement, pyrogener Kieselsäure, insbesondere unbehandelter, polarer pyrogener Kieselsäure, Quarzsand, Quarzmehl, und Mischungen aus zwei oder mehreren davon. Ganz besonders bevorzugt ist für die Reaktivharzkomponente (A) eine Mischung aus Zement (insbesondere Aluminatzement (oft auch als Tonerdezement bezeichnet) oder Portlandzement), pyrogener Kieselsäure und Quarzsand. Für die Härterkomponente (B) ist pyrogene Kieselsäure als alleiniger Füllstoff oder als einer von mehreren Füllstoffen bevorzugt; besonders bevorzugt sind neben der pyrogenen Kieselsäure noch einer oder mehrere weitere Füllstoffe vorhanden.

Als Additive in der Reaktivharzkomponente (A) finden übliche Additive Verwendung, also Thixotropiermittel, wie gegebenenfalls organisch oder anorganisch nachbehandelte pyrogene Kieselsäure (falls sie nicht schon als Füllstoff verwendet wird), insbesondere apolar nachbehandelte pyrogene Kieselsäure, Bentonite, Alkyl- und Methylcellulosen, Rhizinusölderivate oder dergleichen, Weichmacher, wie Phthalsäure- oder Sebacinsäureester, Antistatikmittel, Verdickungsmittel, Flexibilisatoren, Rheologiehilfsmittel, Netzmittel, färbende Zusätze, wie Farbstoffe oder insbesondere Pigmente, beispielsweise zum unterschiedlichen Anfärben der Komponenten zur besseren Kontrolle von deren Durchmischung, oder dergleichen, oder Gemische von zwei oder mehr davon. Auch nicht reaktive Verdünnungsmittel (Lösungsmittel) können, vorzugsweise in einer Menge bis zu 30 Gew.-%, bezogen auf die Gesamtmenge der Reaktivharzkomponente enthalten sein, wie Niederalkylketone, z.B. Aceton, Diniederalkyl-niederalkanoylamide, wie Dimethylacetamid, Niederalkylbenzole, wie Xylole oder Toluol, Phthalsäureester oder Paraffine, Wasser oder Glykole. Ferner können Metallfänger in Form von oberflächenmodifizierten pyrogenen Kieselsäuren in der Reaktivharzkomponente enthalten sein. Bevorzugt ist mindestens ein Thixotropiermittel als Additiv vorhanden, besonders bevorzugt eine organisch oder anorganisch nachbehandelte pyrogene Kieselsäure, ganz besonders bevorzugt eine apolar nachbehandelte pyrogene Kieselsäure, z.B. mit Polydimethylsiloxan (PDMS) nachbehandelte pyrogene Kieselsäure, insbesondere bevorzugt die in den Beispielen verwendete apolar nachbehandelte pyrogene Kieselsäure.

In dieser Hinsicht wird Bezug genommen auf die Anmeldungen WO 2002/079341 A1 und WO 2002/079293 A1 sowie WO 2011/128061 A1.

In einer Ausführungsform kann die Reaktivharzkomponente zusätzlich einen Haftvermittler enthalten. Durch den Einsatz eines Haftvermittlers wird die Vernetzung der Bohrlochwand mit der Dübelmasse verbessert, so dass sich die Haftung im ausgehärteten Zustand erhöht. Dies ist für die Verwendung einer Zweikomponenten-Dübelmasse z.B. in mit einem Diamantbohrer gebohrten Bohrlöchern von Bedeutung und erhöht die Versagensverbundspannung. Geeignete Haftvermittler sind aus der Gruppe der Silane, die mit weiteren reaktiven organischen Gruppen funktionalisiert sind und in das Polymernetzwerk eingebunden werden können, ausgewählt. Diese Gruppe umfasst z.B. 3-(Meth)acryloyloxypropyltrimethoxysilan, 3-(Meth)acryloyloxypropyltriethoxysilan, 3-(Meth)acryloyloxymethyltrimethoxysilan, 3-(Meth)acryloyloxymethyltriethoxysilan, Vinyltrimethoxysilan, Vinyltriethoxysilan, funktionalisiertes Tetraethoxysilan, funktionalisiertes Tetramethoxysilan, funktionalisiertes Tetrapropoxysilan, funktionalisiertes Ethyl- oder Propylpolysilikat, und Gemische von zwei oder mehr davon. In dieser Hinsicht wird Bezug genommen auf die Anmeldung DE 10 2009 059210 A1.

Der Haftvermittler ist zweckmäßig in Mengen von etwa 1 bis etwa 10 Gew.-% bezogen auf das Gesamtgewicht der Reaktivharzkomponente (A) enthalten.

Die vorliegende Erfindung betrifft auch ein Reaktivharz-System. Das erfindungsgemäße Reaktivharz-System ist ein Zwei- oder Mehrkomponenten-System, bevorzugt ein Zweikomponenten-System. Eine der Komponenten ist die erfindungsgemäße Reaktivharzkomponente (A), die andere eine Härterkomponente (B). Letztere enthält einen Initiator, mit dem bei Vermischen der Komponenten die Polymerisation des Reaktivharzes in Gang gesetzt wird.

Bei einer bevorzugten Ausführungsform des erfindungsgemäßen Reaktivharz-Systems ist das Reaktivharz-System ein Zweikomponenten-System und enthält die Reaktivharzkomponente (A) neben dem erfindungsgemäßen Reaktivharz zusätzlich noch eine hydraulisch abbindende oder polykondensierbare anorganische Verbindung, insbesondere Zement, und die Härterkomponente (B) neben dem Initiator für die Polymerisation des Reaktivharzes noch Wasser. Derartige Hybridmörtelsysteme sind ausführlich in DE 4231161 A1 beschrieben. Dabei enthält die Komponente (A) vorzugsweise als hydraulisch abbindende oder polykondensierbare anorganische Verbindung Zement, beispielsweise Portlandzement oder Tonerdezement, wobei übergangsmetalloxidfreie oder übergangsmetallarme Zemente besonders bevorzugt sind. Als hydraulisch abbindende anorganische Verbindung kann auch Gips als solcher oder in

Mischung mit dem Zement eingesetzt werden. Die Komponente (A) kann als polykondensierbare anorganische Verbindung auch silikatische, polykondensierbare Verbindungen, insbesondere lösliches, gelöstes und/oder amorphes Siliziumdioxid enthaltende Stoffe wie z.B. polare, nicht nachbehandelte pyrogene Kieselsäure umfassen.

Des Weiteren ist es bevorzugt, dass die Komponente (A) auch ein Thixotropiermittel enthält, vorzugsweise apolar nachbehandelte pyrogene Kieselsäure, besonders bevorzugt mit Polydimethylsiloxan (PDMS) nachbehandelte pyrogene Kieselsäure, ganz besonders bevorzugt die in den Beispielen verwendete apolar nachbehandelte pyrogene Kieselsäure.

Die erfindungsgemäße Reaktivharzkomponente (A) enthält:

- das wie oben definierte erfindungsgemäße Reaktivharz, bevorzugt eine wie oben beschriebene bevorzugte Ausführungsform davon;
- mindestens eine hydraulisch abbindende oder polykondensierbare anorganische Verbindung, bevorzugt Zement;
- mindestens einen weiteren Füllstoff, bevorzugt Quarzsand; und
- mindestens ein Thixotropiermittel, bevorzugt apolar nachbehandelte pyrogene Kieselsäure.

In einer bevorzugten Ausführungsform enthält die Reaktivharzkomponente (A):

- den erfindungsgemäßen reaktiven Amin-Beschleuniger;
- mindestens ein wie oben definiertes Backbone-Harz, bevorzugt Urethan(meth)acrylat;
- mindestens einen Reaktivverdünner, bevorzugt HPMA und/oder BDDMA;
- mindestens einen wie oben definierten Inhibitor, bevorzugt einen Inhibitor vom Typ Piperidinyl-N-oxyl oder Tetrahydropyrrol-N-oxyl, bevorzugt TEMPOL;
- mindestens eine hydraulisch abbindende oder polykondensierbare anorganische Verbindung, bevorzugt Zement;
- mindestens einen weiteren Füllstoff, bevorzugt Quarzsand; und
- mindestens ein Thixotropiermittel, bevorzugt apolar nachbehandelte pyrogene Kieselsäure.

In einer noch bevorzugteren Ausführungsform enthält die Reaktivharzkomponente (A):

- den erfindungsgemäßen reaktiven Amin-Beschleuniger;
- mindestens ein wie oben definiertes Urethan(meth)acrylat;
- HPMA und/oder BDDMA;
- mindestens einen wie oben definierten Inhibitor vom Typ Piperidinyl-N-oxyl oder Tetrahydropyrrol-N-oxyl, bevorzugt TEMPOL;
- mindestens einen weiteren Inhibitor, der ausgewählt ist aus der Gruppe besteht aus Catecholen und Phenothiazinen;
- Zement; und
- mindestens ein Thixotropiermittel, bevorzugt apolar nachbehandelte pyrogene Kieselsäure.

In einer noch bevorzugteren Ausführungsform enthält die Reaktivharzkomponente (A):

- den erfindungsgemäßen reaktiven Amin-Beschleuniger;
- mindestens ein wie oben definiertes Urethan(meth)acrylat;
- HPMA und/oder BDDMA;
- TEMPOL;
- mindestens einen weiteren Inhibitor, der ausgewählt ist aus der Gruppe besteht aus Catecholen und Phenothiazinen;
- Zement;
- apolar nachbehandelte pyrogene Kieselsäure; und
- Quarzsand.

Die für ein erfindungsgemäßes Reaktivharz-System neben der Reaktivharzkomponente (A) erforderliche Härterkomponente (B) enthält typischerweise:

- mindestens einen Initiator für die Initiierung der Polymerisation des Reaktivharzes, bevorzugt Benzoylperoxid (BPO) oder tert-Butylperoxybenzoat; und
- Wasser.

In einer bevorzugten Ausführungsform enthält die Härterkomponente (B):

- mindestens einen Initiator für die Initiierung der Polymerisation des Reaktivharzes, bevorzugt Benzoylperoxid (BPO)

oder tert-Butylperoxybenzoat;
- mindestens einen Füllstoff, bevorzugt pyrogene Kieselsäure; und
- Wasser.

In einer bevorzugteren Ausführungsform enthält die Härterkomponente (B):

- Benzoylperoxid (BPO) oder tert-Butylperoxybenzoat für die Initiierung der Polymerisation des Reaktivharzes;
- pyrogene Kieselsäure; und
- Wasser.

Die Reaktivharzkomponenten (A) und die Härterkomponenten (B) in jeder dieser Ausführungsformen sind beliebig miteinander kombinierbar.

In einer besonders bevorzugten Ausführungsform sind die Bestandteile des erfindungsgemäßen Reaktivharzes bzw. der erfindungsgemäßen Reaktivharzkomponente eines oder mehrere der Bestandteile, welche in den erfindungsgemäßen Beispielen genannt werden. Ganz besonders bevorzugt sind Reaktivharze bzw. Reaktivharzkomponenten, welche dieselben Bestandteile enthalten oder aus denselben Bestandteilen bestehen, wie sie in den einzelnen erfindungsgemäßen Beispielen genannt werden, und zwar bevorzugt in etwa in den dort genannten Anteilen.

Das den erfindungsgemäßen Amin-Beschleuniger enthaltende erfindungsgemäße Reaktivharz sowie die erfindungsgemäße Reaktivharzkomponente (A), welche dieses Reaktivharz enthält, und das erfindungsgemäße Reaktivharz-System, welches diese Reaktivharzkomponente als eine Komponente umfasst, zeichnen sich dadurch aus, dass der erfindungsgemäße Amin-Beschleuniger dank der olefinischen Gruppen bei der radikalischen Härtung nahezu vollständig im Polymernetzwerk eingebaut wird. Hierdurch wird eine Diffusion der Amin-Beschleuniger an die Oberfläche der gehärteten Werkstoffe weitgehend oder gar ganz verhindert.

Die erfindungsgemäßen Reaktivharze sind in vielen Bereichen, bei denen sonst üblicherweise ungesättigte Polyesterharze, Vinylesterharze oder Vinylesterurethanharze Verwendung finden, einsetzbar. Sie können insbesondere zur Herstellung von Reaktivharzmörteln für bauliche Anwendungen, wie die chemische Befestigung verwendet werden.

Das erfindungsgemäße Reaktivharz findet üblicherweise Verwendung als Harzbestandteil in der Reaktivharzkomponente eines Mehrkomponenten-Systems, typischerweise einem Zweikomponenten-System aus einer Reaktivharzkomponente (A) und einer Härterkomponente (B). Dieses Mehrkomponenten-System kann in Form eines Patronen-Systems, eines Kartuschen-Systems oder eines Folienbeutel-Systems vorliegen. Bei der bestimmungsgemäßen Verwendung des Systems werden die Komponenten entweder unter Einwirkung mechanischer Kräfte oder durch Gasdruck aus den Patronen, Kartuschen oder Folienbeuteln ausgepresst, miteinander vermischt, vorzugsweise mit Hilfe eines Statikmischers, durch den die Bestandteile hindurchgeführt werden, und in das Bohrloch eingeführt, wonach die zu befestigenden Einrichtungen, wie Ankergewindestangen und dergleichen, in das mit dem aushärtenden Reaktivharz beschickte Bohrloch eingebracht und entsprechend justiert werden.

Ein solches Reaktivharz-System findet vor allem im Baubereich Verwendung, etwa zur Instandsetzung von Beton, als Polymerbeton, als Beschichtungsmasse auf Kunstharzbasis oder als kalthärtende Straßenmarkierung. Besonders eignet es sich zur chemischen Befestigung von Verankerungsmitteln, wie Ankern, Bewehrungseisen, Schrauben und dergleichen, in Bohrlöchern, insbesondere in Bohrlöchern in verschiedenen Untergründen, insbesondere mineralischen Untergründen, wie solche auf der Grundlage von Beton, Porenbeton, Ziegelwerk, Kalksandstein, Sandstein, Naturstein, Glas und dergleichen, und metallischen Untergründen, wie solche aus Stahl. In einer Ausführungsform ist der Untergrund des Bohrlochs Beton, und das Verankerungsmittel besteht aus Stahl oder Eisen. In einer weiteren Ausführungsform ist der Untergrund des Bohrlochs Stahl, und das Verankerungsmittel besteht aus Stahl oder Eisen.

Ein weiterer Gegenstand der Erfindung ist die Verwendung des erfindungsgemäßen Reaktivharzes als Bestandteil eines aushärtbaren Bindemittels oder als aushärtbares Bindemittel, insbesondere zur Befestigung von Verankerungsmitteln in Bohrlöchern verschiedenen Untergrunds und zum baulichen Kleben. In einer Ausführungsform ist der Untergrund des Bohrlochs Beton, und das Verankerungsmittel besteht aus Stahl oder Eisen. In einer weiteren Ausführungsform ist der Untergrund des Bohrlochs Stahl, und das Verankerungsmittel besteht aus Stahl oder Eisen. Bevorzugt hat das Stahl-Bohrloch Rillen.

Die Erfindung wird im Folgenden anhand einer Reihe von Beispielen näher erläutert. Alle Beispiele und Abbildungen stützen den Umfang der Ansprüche. Die Erfindung ist jedoch nicht auf die spezifischen, in den Beispielen und Abbildungen gezeigten Ausführungsformen beschränkt.

**BEISPIELE**

Alle hier aufgelisteten Bestandteile der Zusammensetzungen sind - soweit nicht anders angegeben - kommerziell erhältlich und wurden in der kommerziell üblichen Qualität eingesetzt.

Alle in den Beispielen gemachten %- und ppm-Angaben beziehen sich auf das Gesamtgewicht der beschriebenen Zusammensetzung als Kalkulationsbasis, soweit nicht anders angegeben.

**Herstellungsbeispiel 1: Herstellung des reaktiven Amin-Beschleunigers**

**Aus primären Anilinen und monomerem Bisphenol-A-diglycidylether:**

1 Äq. Bisphenol-A-diglycidylether (Epilox® A 19-03; Epoxyäquivalentgewicht 183 g/mol; LEUNA-Harze GmbH) wurde vollständig im Rundkolben vorgelegt, mit 0,5 Äq. eines primären Anilins, 1,1 Äq. Methacrylsäure (BASF SE), 0,4 Gew.% Tetraethylammoniumbromid (Merck KGaA) sowie 230 ppm Tempol (Evonik Industries AG) und 160 ppm Phenothiazin (Allessa GmbH) versetzt und auf 100 °C temperiert. Es wurde gerührt bis nach etwa 4h über Dünnschichtchromatographie (stationäre Phase: Kieselgelplatte; Eluent: Petrolether:Ethylacetat 1:1) vollständiger Umsatz angezeigt wurde, also kein freies Amin mehr nachweisbar war.

Es wurde mit 20 Gew.% Hydroxypropylmethacrylat (HPMA, Evonik Industries AG) verdünnt, mit 400 ppm Tempol nachstabilisiert und abgekühlt.

Um die Viskosität zu erniedrigen, wurde bei der Verwendung von *para*-Toluidin als primärem Anilin vor Zugabe des Anilins der Diglycidylether partiell mit 0,5 Äq. Methacrylsäure bei 80°C eine Stunde umgesetzt.

| **Eingesetztes primäres Anilin** | **Lieferant** | **Abkürzung für den resultierenden Amin-Beschleuniger** |
|---|---|---|
| *meta*-Toluidin | Alfa Aesar | mT |
| *para*-Toluidin | Sigma-Aldrich Chemie GmbH | pT |
| *para*-Bromanilin | TCI Deutschland GmbH | pBrA |
| *para*-Chloranilin | TCI Deutschland GmbH | pClA |
| *meta*-Chloro-*para*-methylanilin | TCI Deutschland GmbH | 3Cl4MeA |
| *para*-tert-Butylanilin | TCI Deutschland GmbH | ptBuA |

**Aus sekundären Anilinen und monomerem Bisphenol-A-diglycidylether:**

1 Äq. Bisphenol-A-diglycidylether (Epilox® A 19-03; Epoxyäquivalentgewicht 183 g/mol; LEUNA-Harze GmbH) wurde vollständig im Rundkolben vorgelegt, mit 1 Äq. eines sekundären Anilins, 1,1 Äq. Methacrylsäure (BASF SE), 0,4 Gew.% Tetraethylammoniumbromid (Merck KGaA) sowie 230 ppm Tempol (Evonik Industries AG) und 160 ppm Phenothiazin (Allessa GmbH) versetzt und auf 100 °C temperiert. Es wurde gerührt bis nach etwa 4h über Dünnschichtchromatographie (stationäre Phase: Kieselgelplatte; Eluent: Petrolether:Ethylacetat 1:1) vollständiger Umsatz angezeigt wurde, also kein freies Amin mehr nachweisbar war.

Es wurde mit 20 Gew% HPMA (Evonik Industries AG) verdünnt, mit 400 ppm Tempol nachstabilisiert und abgekühlt.

**Aus sekundären Anilinen und polymerem Bisphenol-A-diglycidylether:**

0,5 Äq. Bisphenol-A-diglycidylether (Epilox® A 19-03; Epoxyäquivalentgewicht 183 g/mol; LEUNA-Harze GmbH) und 0,4 Äq. Bisphenol-A-diglycidylether (Epilox® A 50-02; Epoxyäquivalentgewicht 485 g/mol; LEUNA-Harze GmbH) wurden vollständig im Rundkolben vorgelegt, mit 0,9 Äq. eines sekundären Anilins, 1 Äq. Methacrylsäure (BASF SE), 0,4 Gew.% Tetraethylammoniumbromid (Merck KgaA) sowie 230 ppm Tempol (Evonik Industries AG) und 160 ppm Phenothiazin (Allessa GmbH) versetzt und auf 100 °C temperiert. Es wurde gerührt bis nach etwa 4h über Dünnschichtchromatographie vollständiger Umsatz angezeigt wurde.

Es wurde mit 20 Gew.% HPMA (Visiomer® HPMA 98, Evonik Industries AG) verdünnt, mit 400 ppm Tempol nachstabilisiert und abgekühlt.

| **Eingesetztes sekundäres Anilin** | **Lieferant** | **Abkürzung für den resultierenden Amin-Beschleuniger ("poly" steht für Beschleuniger, die mit polymerem Bisphenol-A-diglycidylether hergestellt wurden)** |
|---|---|---|
| N-Methyl-p-toluidin | TCI Deutschland GmbH | NMepT bzw. polyNMepT |
| N-Ethyl-p-toluidin | TCI Deutschland GmbH | NEtpT bzw. polyNEtpT |
| N-Ethyl-m-toluidin | TCI Deutschland GmbH | NEtmT bzw. polyNEtmT |
| N-Ethylanilin | TCI Deutschland GmbH | NEtA |
| N-(2-Hydroxyethyl)anilin | TCI Deutschland GmbH | NHOEtA |
| N-Dodecylanilin | TCI Deutschland GmbH | NdodecA |
| N-Ethyl-para-chloroanilin | TCI Deutschland GmbH | NEtpCIA |

**Herstellungsbeispiel 2: Herstellung eines Reaktivharzes**

Ein gemäß Beispiel 1 hergestellter Amin-Beschleuniger bzw., als Vergleich, DiPpT wurde in einer Menge entsprechend 7,8 mmol Amin/100g Harzmischung ($c_{ges}$ = 7,8 mmol/100g Harz) mit 15,13 Gew.% HPMA (Visiomer® HPMA 98, Evonik Industries AG), 32,75 Gew.% 1,4-Butandioldimethacrylat (Visiomer® 1,4-BDDMA, Evonik Industries AG), 0,25 Gew.% TBC (tert-Butylbrenzcatechin, Rhodia), 0,015 Gew.% Tempol (Evonik Industries AG) und ad 100% (Menge wurde angepasst, so dass Summe aller Harzanteile 100% ergab) UMA/HPMA-Reaktivharz-Masterbatch (hergestellt analog EP 0 713 015 A1, Beispiel A3, wobei die Mengen so angepasst wurden, dass 65 Gew.-% Urethanmethacrylatharz in 35 Gew.% HPMA erhalten wurden) kombiniert.

**Testbeispiel 1: Gelzeit und maximale Reaktivitätstemperatur**

Die **Gelzeit** wurde wie folgt ermittelt:
Gemessen wurde die Gelzeit (für das Reaktivharz mit $t_{hg25°C}$ bezeichnet) ausgedrückt als die Zeitspanne gemessen vom Zeitpunkt der Zugabe eines Initiators zur Initialisierung der Härtung an bis zu dem Zeitpunkt, bei dem die Zusammensetzung eine Temperatur von 50°C erreicht hat. Die Messung erfolgte wie folgt:

Die Gelzeit nach Zugabe des Initiators (Perkadox® 20S (Akzo), Gewichtsverhältnis Reaktivharz:Initiator 100:30) zum gemäß Herstellungsbeispiel 2 hergestellten Reaktivharz wurde mit einem herkömmlichen Gerät (Geltimer, Fa. WKS Informatik) bei einer Starttemperatur von 25°C bestimmt. Die Mischung wurde hierzu nach Zugabe des Initiators bis zu einer Höhe von 4 cm unterhalb des Randes in ein Teströhrchen gefüllt, wobei das Teströhrchen bei einer Temperatur von 25°C gehalten wurde (DIN 16945, DIN EN ISO 9396). Ein Glasstab oder eine Spindel wurde mit 10 Hüben pro Minuten in der Mischung auf und ab bewegt. Die Gelzeit entspricht der Zeitspanne nach Zugabe des Initiators, nach der eine Temperatur von 50°C in der Mischung gemessen wurde.

Die **maximale Reaktivitätstemperatur $T_{max}$** entspricht dem Maximum der Temperaturkurve in der Gelzeitmessung. Um dieses Maximum zu ermitteln, wurde die Gelzeitmessung nach Erreichen der Temperatur von 50°C solange fortgeführt, bis das Maxium der Temperaturkurve überschritten war.

Die Ergebnisse sind in der folgenden Tabelle wiedergegeben:

| Beschleuniger aus Amin | Gelzeit: $t_{hg,25°C}$ | Maximale Reaktivitätstemperatur: $T_{max}$ |
|---|---|---|
| NMepT | 2,40 min | 168°C |
| N EtpT | 3,70 min | 162°C |
| NEtmT | 9 min | 164°C |
| mT | 21 min | 162°C |
| pT | 5,4 min | 163°C |
| NEtA | 22 min | 162°C |
| NHOEtA | 33 min | 159°C |
| pBrA | 100 min | 156°C |
| ptBuA | 11 min | 157°C |

| Beschleuniger aus Amin | Gelzeit: $t_{hg,25°C}$ | Maximale Reaktivitätstemperatur: $T_{max}$ |
|---|---|---|
| NdodecA | 20 min | 158°C |
| pClA | 72min | 155°C |
| 3Cl4MeA | 36min | 155°C |
| NEtpCl | 42min | 158°C |
| (1:1) NMepT+pBrA | 7,25 min | 165-167°C |
| (2:1) NMepT+pBrA | 4,15 min | 165°C |
| (1:1) NEtpT+pBrA | 21 min | 156°C |
| (1:1:1) NMepT+NEtmT+pBrA | 5,7 min | 159-163°C |
| (50%, also Hälfte der Beschleunigermenge) NmepT | 6,8 min | 167-168°C |
| (1:1) NMepT+NEtA | 6,0 min | 163-165°C |
| (1:1) NMepT+NEtmT | 5,3 min | 164°C |
| (2:1) NMepT+NEtmT | 3,92 min | 160°C |
| (3:2) NMepT+NEtmT | 4,16 min | 158°C |
| NMepT+pBrA (3:2) | 6,22 min | 158°C |
| polyNMepT | 2,30 min | 157°C |
| polyNEtpT | 3,60 min | 161°C |
| polyNEtmT | 9 min | 162°C |
| polyNMepT+ NEtA (1:1) | 5,08 min | 159°C |
| polyNMepT+NEtmT (3:2) | 3,30 min | 164°C |
| | | |
| | | |
| Monomer DiPpT (Referenz) | 4,9 min | 160°C |
| UMA-gebundenes DiPpT (hergestellt gemäß WO 12/164020) | 29 min | 154°C |

**Folgerungen aus diesen Tests:**

Durch strukturelle Änderung am Stickstoff oder am aromatische Ring des Anilins bzw. des Toluidins kann die beschleunigende Wirkung des Anilins bzw. Toluidins stark verändert werden.

Unabhängig von der Gelzeit zeigten alle reaktiven Amin-Beschleuniger hohe Peaktemperaturen (maximale Reaktivitätstemperaturen), was auf eine sehr gute Aushärtung hindeutet.

**Herstellungsbeispiel 3: Reaktivharzkomponente (A)**

In 39,3 Gew.% eines gemäß Herstellungsbeispiel 2 hergestellten Reaktivharzes wurden in einem Dissolver unter Vakuum 37,7 Gew.% Quarzsand F32 (Quarzwerke Frechen), 20,5 Gew.% Aluminatzement Secar® 80 (Kerneos) und 2,5 Gew.% apolar nachbehandelte pyrogene Kieselsäure Cab-O-Sil® 720 (Cabot Rheinfelden) dispergiert. Die Gelzeit der Reaktivharzkomponente, $t_{mg,25°C}$, wurde gemessen mit demselben Verfahren wie in Testbeispiel 1 beschrieben, wobei statt des Reaktivharzes aus Herstellungsbeispiel 2 die hier beschriebene Reaktivharzkomponente getestet wurde.

| **Bezeichnung** | **Beschleuniger (molares Verhältnis) mit $c_{ges}$ = 7,8 mmol/100g Harz** | **Gelzeit: $t_{mg,25°C}$ [min]** |
|---|---|---|
| Vergleichsbeispiel1 | DiPpT | 4,50 |
| Beispiel1 | NMepT/NEtmT (3/2) | 4,10 |
| Beispiel2 | NMepT/pBrA (3/2) | 6,60 |
| Beispiels | NMepT/pBrA (2/1) | 4,20 |
| Beispiel4 | polyNMepT/NEtmT (3/2) | 3,52 |

**Testbeispiel 2: Messung der Verbundspannung**

Ein Reaktivharzsystem aus einer gemäß Herstellungsbeispiel 3 hergestellten Reaktivharzkomponente (A) und der als Härterkomponente (B) eingesetzten kommerziellen Härterkomponente HY-110 B (Hilti) wurde in eine Plastikkartusche (Firma Ritter GmbH; Volumenverhältnis A:B = 3:1) mit den Innendurchmessern 47 mm (Komponente (A)) bzw. 28 mm (Komponente (B)) gefüllt und wie folgt getestet:

Zur Bestimmung der mit Reaktivharzsystemen gemäß dem Vergleichsbeispiel 1 und gemäß Beispiel 1 bis Beispiel 4 erzielten Scherfestigkeit (Synonym: Verbundspannung) wurde das gemischte Reaktivharzsystem (also die Mischung aus Reaktivharzkomponente (A) und Härterkomponente (B) im Volumenverhältnis A:B = 3:1) in eine Stahlhülse mit definierter Geometrie und definierter Füllhöhe des Mörtels (Einbindetiefe) eingebracht. Anschließend wurde eine Ankerstange mittels einer Zentrierhilfe mittig in der mit der Mischung gefüllten Stahlhülse platziert. Nach Aushärtung bei 25°C und für mindestens 12 Stunden wurde die Probe mit Hilfe eines Gewindeadapters in eine Zugprüfmaschine eingeschraubt (Fabrikat: Zwick Roell Z050, 50 kN). Die Probe wurde mit Zugkraft bei definierter Geschwindigkeit bis zum Versagen belastet. Die entsprechende Kraft-Weg-Abhängigkeit wurde kontinuierlich registriert. Es wurden jeweils fünf Einzelmessungen durchgeführt und der Mittelwert der maximalen Kraft beim Versagen berechnet.

Zur Durchführung der Messungen wurden Ankerstangen mit Gewinde M8 und Stahlhülsen mit folgender Geometrie verwendet:

| | |
|---|---|
| Hinterschnitttiefe: | 0,35+/-0,02 mm |
| Hinterschnittbreite: | 2 mm |
| Einbindetiefe: | 36 mm |
| Innendurchmesser: | 12 mm |

Die aus diesen Messungen ermittelte Scherfestigkeit ist definiert als der Quotient aus maximaler Kraft beim Versagen und der Scherfläche der verwendeten Ankerstange (Ankerstange M8: 904,3 $mm^2$). Die Ergebnisse der Messungen sind in der folgenden Tabelle wiedergegeben:

| | Vergleichsbeispiel 1 | Beispiel 1 | Beispiel 2 | Beispiel 3 | Beispiel 4 |
|---|---|---|---|---|---|
| Verbundspannun g [$N/mm^2$] | 16,5±1,3 | 14,2±2,2 | 15,2±2,0 | 12,5±1,0 | 14,3±1,3 |

**Folgerung:**

Ausgehärtete Reaktivharzsysteme mit unterschiedlichen reaktiven Amin-Beschleunigern (Beispiel1 bis Beispiel4) zeigten eine vergleichbare Verbundspannung wie Reaktivharzsysteme, die als Beschleuniger DiPpT enthielten (Vergleichsbeispiel1).

**Testbeispiel 3: Sedimentation**

Ein Vergleich der Sedimentationseigenschaften wurde mit Hilfe eines beschleunigten Tests durchgeführt. Hierfür kam das Instrument Lumifuge® der Firma L.U.M. GmbH zum Einsatz. Folgende Parameter wurden für die Methode verwendet:

| | |
|---|---|
| Lichtfaktor | 0,5 |
| Umdrehungsgeschwindigkeit | 2055 rpm |
| Polyamidküvette | 10 mm |
| Temperatur | 35°C |
| 8 Kanäle (Parallelmessungen) | |
| Zeit | 255 Messungen alle 20s |
| Modell | 1120-28 |
| Füllmenge Küvette | 1,3 mL |

Folgende Reaktivharzkomponenten (A) wurden getestet:

| Beispiel | Beschleuniger (molares Verhältnis) mit $c_{ges}$ = 7,8 mmol/100g Harz |
|---|---|
| Vergleichsbeispiel1 | DiPpT |
| Beispiel5 | NEtpT/NEtmT/pBrA (1/1/1) |
| Beispiele | pBrA |

Folgende Messergebnisse (Mittelwerte aus 8 Parallelmessungen) wurden erzielt:

| | Vergleichsbeispiel1 | Beispiel5 | Beispiel6 |
|---|---|---|---|
| SedimentationsGeschwindigkeit [μm/s] | 0,477±0,04 | 0,328±0,03 | 0,338±0,02 |

**Folgerung:**

Beispiel 5 und Beispiel 6 zeigten eine um den Faktor 1,4 verlangsamte Sedimentationsgeschwindigkeit und somit verbesserter Lagerfähigkeit gegenüber dem Vergleichsbeispiel 1.

**Patentansprüche**

**1.** Beschleuniger für ein Reaktivharz-System, hergestellt durch Reaktion der folgenden Komponenten:

(1) aromatisches primäres oder sekundäres Amin, oder eine Mischung davon;
(2) Diglycidylether eines Diols oder Polyols mit 2 bis 20 C-Atomen; und
(3) $\alpha,\beta$-ungesättigte Carbonsäure ausgewählt aus der Gruppe bestehend aus verzweigten und unverzweigten $C_2$-$C_{10}$-$\alpha,\beta$-ungesättigte Carbonsäuren,
wobei die Öffnung der im Diglycidylether vorhanden Epoxidgruppen zur Ausbildung von Glycerin-Brücken zwischen dem Amin und dem Diol und zwischen der $\alpha,\beta$-ungesättigten Carbonsäure und dem Diol führt, was den fertigen Beschleuniger ergibt,
wobei das aromatische primäre oder sekundäre Amin ausgewählt ist aus der Gruppe von aromatischen primären oder sekundären Aminen, in welchen der Aromatenrest entweder unsubstituiert ist, oder substituiert ist mit einem oder mehreren Substituenten $R^1$ ausgewählt aus der Gruppe bestehend aus Halogen, Pseudohalogen,

$C_1$-$C_{20}$-Alkyl, Hydroxy-$C_1$-$C_{20}$-Alkyl, $C_2$-$C_{20}$-Alkenyl, Hydroxy-$C_2$-$C_{20}$-Alkenyl, $C_2$-$C_{20}$-Alkinyl, Hydroxy-$C_2$-$C_{20}$-Alkinyl und Phenyl.

2. Beschleuniger für ein Reaktivharz-System gemäß Anspruch 1, wobei das aromatische primäre oder sekundäre Amin ein primäres Amin ist, welches ausgewählt ist aus der Gruppe bestehend aus einem unsubstituierten Toluidin, einem am Aromaten halogenierten Toluidin ohne weiteren Substituenten, und einem am Aromaten halogenierten Toluidin, welches am Aromaten eine weitere $C_1$-$C_4$-Alkylgruppe trägt und wobei der Diglycidylether ein Diglycidylether eines Diols ist.

3. Beschleuniger für ein Reaktivharz-System gemäß Anspruch 1, wobei das aromatische primäre oder sekundäre Amin ein sekundäres Amin ist, welches ausgewählt ist aus der Gruppe bestehend aus einem unsubstituierten Anilin oder Toluidin, einem am Aromaten halogenierten Toluidin oder Anilin ohne weiteren Substituenten, und einem am Aromaten halogenierten Toluidin oder Anilin, welches am Aromaten eine weitere $C_1$-$C_4$-Alkylgruppe trägt.

4. Beschleuniger für ein Reaktivharzsystem gemäß irgendeinem der Ansprüche 1 bis 3, wobei das Diol ausgewählt ist aus der Gruppe bestehend aus Bisphenolen, Neopentylglycol, Ethylenglycol, Phenol-Novolac-Harz, Cresol-Novolac-Harz, und 1,4-Butandiol.

5. Beschleuniger für ein Reaktivharzsystem gemäß irgendeinem der Ansprüche 1 bis 4, wobei die $\alpha,\beta$-ungesättigte Carbonsäure ausgewählt ist aus der Gruppe bestehend aus Tiglinsäure, Sorbinsäure, Crotonsäure, Methacrylsäure und Acrylsäure.

6. Beschleuniger für ein Reaktivharzsystem gemäß irgendeinem der Ansprüche 1 bis 5, wobei das aromatische Amin ein primäres aromatisches Amin ist und wobei das Äquivalent-Verhältnis Diglycidylether: $\alpha,\beta$-ungesättigte Carbonsäure: primäres aromatisches Amin im Bereich von etwa 1 : 0,3 : 0,8 bis etwa 1 : 2,09 : 0,01 liegt.

7. Beschleuniger für ein Reaktivharzsystem gemäß irgendeinem der Ansprüche 1 bis 5, wobei das aromatische Amin ein sekundäres aromatisches Amin ist und wobei das Äquivalent-Verhältnis Diglycidylether: $\alpha,\beta$-ungesättigte Carbonsäure : sekundäres aromatisches Amin im Bereich von etwa 1 : 0,1 : 2 bis etwa 1 : 2,09 : 0,01 liegt.

8. Beschleuniger für ein Reaktivharz-System gemäß irgendeinem der Ansprüche 1 bis 7, mit der Formel (I):

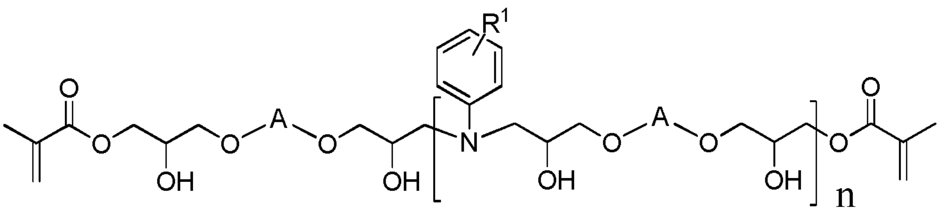

(I),

worin

kein, ein oder mehrere $R^1$ als Substituenten am Phenylring vorhanden sind;
$R^1$ ausgewählt ist aus der Gruppe bestehend aus Halogen, Pseudohalogen, $C_1$-$C_{20}$-Alkyl, Hydroxy-$C_1$-$C_{20}$-Alkyl, $C_2$-$C_{20}$-Alkenyl, Hydroxy-$C_2$-$C_{20}$-Alkenyl, $C_2$-$C_{20}$-Alkinyl, Hydroxy-$C_2$-$C_{20}$-Alkinyl und Phenyl;
n eine ganze Zahl von 1 bis 20, bevorzugt von 1 bis 7, besonders bevorzugt von 1 bis 3 ist; und
A der Rest des Diols ist, welches in dem Diglycidylether (2) enthalten ist;

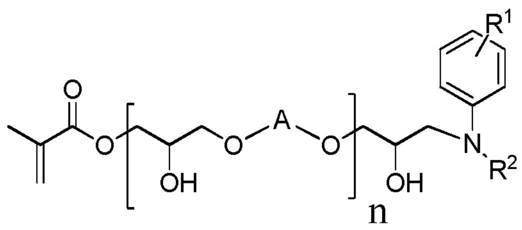

(II),

worin

kein, ein oder mehrere $R^1$ als Substituenten am Phenylring vorhanden sind;
$R^1$ ausgewählt ist aus der Gruppe bestehend aus Halogen, Pseudohalogen, $C_1$-$C_{20}$-Alkyl, Hydroxy-$C_1$-$C_{20}$-Alkyl, $C_2$-$C_{20}$-Alkenyl, Hydroxy-$C_2$-$C_{20}$-Alkenyl, $C_2$-$C_{20}$-Alkinyl, Hydroxy-$C_2$-$C_{20}$-Alkinyl und Phenyl;
$R^2$ ausgewählt ist aus der Gruppe bestehend aus $C_1$-$C_{20}$-Alkyl, Hydroxy-$C_1$-$C_{20}$-Alkyl, $C_2$-$C_{20}$-Alkenyl, Hydroxy-$C_2$-$C_{20}$-Alkenyl, $C_2$-$C_{20}$-Alkinyl, Hydroxy-$C_2$-$C_{20}$-Alkinyl und Aryl;
n eine ganze Zahl von 1 bis 20, bevorzugt von 1 bis 5, besonders bevorzugt von 1 bis 1,5 ist; und
A der Rest des Diols ist, welches in dem Diglycidylether (2) enthalten ist.

**9.** Beschleuniger für ein Reaktivharz-System gemäß Anspruch 8, worin $R^1$ ausgewählt ist aus der Gruppe bestehend aus Halogen, Hydroxy-$C_1$-$C_{20}$-Alkyl und $C_1$-$C_{20}$-Alkyl.

**10.** Beschleuniger für ein Reaktivharz-System gemäß Anspruch 9, worin $R^1$ ausgewählt ist aus der Gruppe bestehend aus Chlor, Brom und $C_1$-$C_6$-Alkyl.

**11.** Beschleuniger für ein Reaktivharz-System gemäß irgendeinem der Ansprüche 1 bis 10, worin der Diglycidylether ein Digylcidylether von Diolen ausgewählt aus der Gruppe bestehend aus Bisphenolen, insbesondere Bisphenol A, Bisphenol F, und Bisphenol S, Neopentylglycol, Ethylenglycol, Phenol-Novolac-Harz, Cresol-Novolac-Harz, und 1,4-Butandiol ist.

**12.** Reaktivharz, enthaltend einen Beschleuniger gemäß irgendeinem der Ansprüche 1 bis 11.

**13.** Reaktivharzkomponente (A) für ein Reaktivharz-System, enthaltend ein Reaktivharz gemäß Anspruch 12.

**14.** Reaktivharz-System mit einer Reaktivharzkomponente (A) gemäß Anspruch 13, und einer Härterkomponente (B), die einen Initiator enthält.

**15.** Reaktivharz-System nach Anspruch 14, wobei mindestens eine der Komponenten (A) oder (B) einen anorganischen Füllstoff enthält.

**16.** Reaktivharz-System nach einem der Ansprüche 14 oder 15, wobei die Reaktivharzkomponente (A) enthält:

- mindestens einen Beschleuniger gemäß irgendeinem der Ansprüche 1 bis 11;
- mindestens ein Backbone-Harz;
- mindestens einen Reaktivverdünner;
- mindestens einen Inhibitor;
- mindestens eine hydraulisch abbindende oder polykondensierbare anorganische Verbindung; und
- mindestens ein Thixotropiermittel,

und die Härterkomponente (B) enthält:

- mindestens einen Initiator für die Initiierung der Polymerisation des Reaktivharzes;
- mindestens einen Füllstoff; und
- Wasser.

**17.** Verwendung eines Reaktivharz-Systems nach einem der Ansprüche 14 bis 16 zur chemischen Befestigung von Verankerungsmitteln in Bohrlöchern oder zum baulichen Kleben.

**18.** Verwendung eines Beschleunigers wie in einem der Ansprüche 1 bis 11 definiert als Beschleuniger in einem Reaktivharz.

## Claims

**1.** Accelerator for a reactive resin system, produced by reacting the following components:

(1) aromatic primary or secondary amine, or a mixture thereof;
(2) diglycidyl ether of a diol or polyol having 2 to 20 C atoms; and
(3) α,β-unsaturated carboxylic acid selected from the group consisting of branched and unbranched $C_2$-$C_{10}$ α,β-unsaturated carboxylic acids,
wherein opening the epoxide groups present in the diglycidyl ether leads to the formation of glycerol bridges between the amine and the diol and between the α,β-unsaturated carboxylic acid and the diol, which results in the finished accelerator,
wherein the aromatic primary or secondary amine is selected from the group of aromatic primary or secondary amines in which the aromatic functional group is either unsubstituted, or is substituted by one or more substituents $R^1$ selected from the group consisting of halogen, pseudohalogen, $C_1$-$C_{20}$ alkyl, hydroxy $C_1$-$C_{20}$ alkyl, $C_2$-$C_{20}$ alkenyl, hydroxy $C_2$-$C_{20}$ alkenyl, $C_2$-$C_{20}$ alkynyl, hydroxy $C_2$-$C_{20}$ alkynyl and phenyl.

**2.** Accelerator for a reactive resin system according to claim 1, wherein the aromatic primary or secondary amine is a primary amine selected from the group consisting of an unsubstituted toluidine, a toluidine halogenated on the aromatic without further substituents, and a toluidine halogenated on the aromatic which carries a further $C_1$-$C_4$ alkyl group on the aromatic, and wherein the diglycidyl ether is a diglycidyl ether of a diol.

**3.** Accelerator for a reactive resin system according to claim 1, wherein the aromatic primary or secondary amine is a secondary amine selected from the group consisting of an unsubstituted aniline or toluidine, a toluidine or aniline halogenated on the aromatic without further substituents, and a toluidine or aniline halogenated on the aromatic which carries a further aromatic $C_1$-$C_4$ alkyl group.

**4.** Accelerator for a reactive resin system according to any of claims 1 to 3, wherein the diol is selected from the group consisting of bisphenols, neopentyl glycol, ethylene glycol, phenol novolac resin, cresol novolac resin, and 1,4-butanediol.

**5.** Accelerator for a reactive resin system according to any of claims 1 to 4, wherein the α,β-unsaturated carboxylic acid is selected from the group consisting of tiglic acid, sorbic acid, crotonic acid, methacrylic acid, and acrylic acid.

**6.** Accelerator for a reactive resin system according to any of claims 1 to 5, wherein the aromatic amine is a primary aromatic amine, and wherein the equivalent ratio of diglycidyl ether: α,β-unsaturated carboxylic acid: primary aromatic amine is in the range of approximately 1 : 0.3 : 0.8 to approximately 1 : 2.09 : 0.01.

**7.** Accelerator for a reactive resin system according to any of claims 1 to 5, wherein the aromatic amine is a secondary aromatic amine, and wherein the equivalent ratio of diglycidyl ether: α,β-unsaturated carboxylic acid: secondary aromatic amine is in the range of approximately 1 : 0.1 : 2 to approximately 1 : 2.09 : 0.01.

**8.** Accelerator for a reactive resin system according to any of claims 1 to 7, having the formula (I):

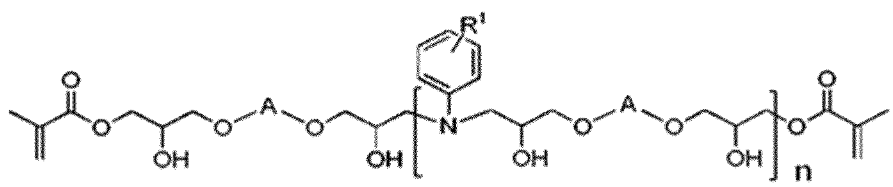

(I),

where

no, one or more $R^1$ are present as substituents on the phenyl ring;
$R^1$ is selected from the group consisting of halogen, pseudohalogen, $C_1$-$C_{20}$ alkyl, hydroxy $C_1$-$C_{20}$ alkyl, $C_2$-$C_{20}$ alkenyl, hydroxy $C_2$-$C_{20}$ alkenyl, $C_2$-$C_{20}$ alkynyl, hydroxy $C_2$-$C_{20}$ alkynyl and phenyl;
n is an integer from 1 to 20, preferably from 1 to 7, particularly preferably from 1 to 3; and
A is the functional group of the diol which is contained in the diglycidyl ether (2);

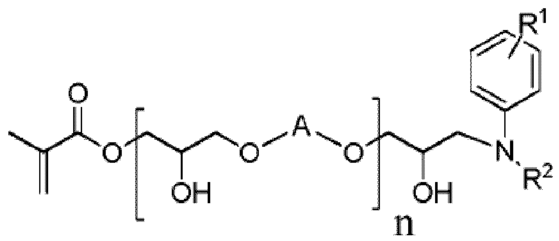

(II),

where

no, one or more $R^1$ are present as substituents on the phenyl ring;
$R^1$ is selected from the group consisting of halogen, pseudohalogen, $C_1$-$C_{20}$ alkyl, hydroxy $C_1$-$C_{20}$ alkyl, $C_2$-$C_{20}$ alkenyl, hydroxy $C_2$-$C_{20}$ alkenyl, $C_2$-$C_{20}$ alkynyl, hydroxy $C_2$-$C_{20}$ alkynyl and phenyl;
$R^2$ is selected from the group consisting of $C_1$-$C_{20}$ alkyl, hydroxy $C_1$-$C_{20}$ alkyl, $C_2$-$C_{20}$ alkenyl, hydroxy $C_2$-$C_{20}$ alkenyl, $C_2$-$C_{20}$ alkynyl, hydroxy $C_2$-$C_{20}$ alkynyl and aryl;
n is an integer from 1 to 20, preferably from 1 to 5, particularly preferably from 1 to 1.5; and
A is the functional group of the diol which is contained in the diglycidyl ether (2).

**9.** Accelerator for a reactive resin system according to claim 8, where $R^1$ is selected from the group consisting of halogen, hydroxy $C_1$-$C_{20}$ alkyl and $C_1$-$C_{20}$ alkyl.

**10.** Accelerator for a reactive resin system according to claim 9, where $R^1$ is selected from the group consisting of chlorine, bromine and $C_1$-$C_6$ alkyl.

**11.** Accelerator for a reactive resin system according to any of claims 1 to 10, where the diglycidyl ether is a diglycidyl ether of diols selected from the group consisting of bisphenols, in particular bisphenol A, bisphenol F, and bisphenol S, neopentyl glycol, ethylene glycol, phenol novolac resin, cresol novolac resin, and 1,4-butanediol.

**12.** Reactive resin, containing an accelerator according to any of claims 1 to 11.

**13.** Reactive resin component (A) for a reactive resin system containing a reactive resin according to claim 12.

**14.** Reactive resin system comprising a reactive resin component (A) according to claim 13 and a hardener component (B) containing an initiator.

**15.** Reactive resin system according to claim 14, wherein at least one of the components (A) or (B) contains an inorganic filler.

**16.** Reactive resin system according to claim 14 or 15, wherein the reactive resin component (A) contains:

- at least one accelerator according to any of claims 1 to 11;
- at least one backbone resin;
- at least one reactive diluent;
- at least one inhibitor;
- at least one hydraulically setting or polycondensable inorganic compound; and
- at least one thixotropic agent,

and the hardener component (B) contains:

- at least one initiator for initiating the polymerization of the reactive resin;
- at least one filler; and

- water.

**17.** Use of a reactive resin system according to any of claims 14 to 16 for chemically fastening anchoring means in boreholes or for structural bonding.

**18.** Use of an accelerator as defined in any of claims 1 to 11 as an accelerator in a reactive resin.

## Revendications

**1.** Accélérateur pour un système de résine réactive, obtenu par mise en réaction des composants suivants :

(1) amine aromatique primaire ou secondaire, ou un mélange de celles-ci ;
(2) éther diglycidylique d'un diol ou d'un polyol comportant 2 à 20 atomes de carbone ; et
(3) acide carboxylique insaturé $\alpha,\beta$ choisi dans le groupe constitué d'acides carboxyliques $\alpha,\beta$ en $C_2$-$C_{10}$ ramifiés et non ramifiés,

dans lequel l'ouverture des groupes époxyde présents dans l'éther diglycidylique conduit à la formation de ponts glycérol entre l'amine et le diol et entre l'acide carboxylique insaturé $\alpha,\beta$ et le diol, ce qui donne l'accélérateur fini,

dans lequel l'amine aromatique primaire ou secondaire est choisie dans le groupe des amines aromatiques primaires ou secondaires, dans lesquels le résidu aromatique est soit non substitué soit substitué par un ou plusieurs substituants $R^1$ choisis dans le groupe constitué d'un halogène, pseudohalogène, alkyle en $C_1$-$C_{20}$, alkyle hydroxy en $C_1$-$C_{20}$, alcényle en $C_2$-$C_{20}$, alcényle hydroxy en $C_2$-$C_{20}$, alcynyle en $C_2$-$C_{20}$, alcynyle hydroxy en $C_2$-$C_{20}$ et phényle.

**2.** Accélérateur pour un système de résine réactive selon la revendication 1, dans lequel l'amine aromatique primaire ou secondaire est une amine primaire qui est choisie dans le groupe constitué d'une toluidine non substituée, toluidine halogénée sur le noyau aromatique sans substituants supplémentaires et toluidine halogénée sur le noyau aromatique, qui porte sur le noyau aromatique un groupe alkyle en $C_1$-$C_4$ supplémentaire, et dans lequel l'éther diglycidylique est un éther diglycidylique d'un diol.

**3.** Accélérateur pour un système de résine réactive selon la revendication 1, dans lequel l'amine aromatique primaire ou secondaire est une amine secondaire qui est choisie dans le groupe constitué d'une aniline ou une toluidine non substituée, toluidine ou aniline halogénée sur le noyau aromatique sans substituants supplémentaires et toluidine ou aniline halogénée sur le noyau aromatique, qui porte sur le noyau aromatique un groupe alkyle en $C_1$-$C_4$ supplémentaire.

**4.** Accélérateur pour un système de résine réactive selon l'une quelconque des revendications 1 à 3, dans lequel le diol est choisi dans le groupe constitué de bisphénols, néopentylglycol, éthylène glycol, résine novolaque phénolique, résine novolaque crésolique et butanediol-1,4.

**5.** Accélérateur pour un système de résine réactive selon l'une quelconque des revendications 1 à 4, dans lequel l'acide carboxylique insaturé $\alpha,\beta$ est choisi dans le groupe constitué d'acide tiglique, acide sorbique, acide crotonique, acide méthacrylique, acide acrylique.

**6.** Accélérateur pour un système de résine réactive selon l'une quelconque des revendications 1 à 5, dans lequel l'amine aromatique est une amine aromatique primaire et dans lequel le rapport d'équivalence éther diglycidylique:acide carboxylique insaturé $\alpha,\beta$:amine aromatique primaire se situe dans la plage d'environ 1:0,3:0,8 à environ 1:2,09:0,01.

**7.** Accélérateur pour un système de résine réactive selon l'une quelconque des revendications 1 à 5, dans lequel l'amine aromatique est une amine aromatique secondaire et dans lequel le rapport d'équivalence éther diglycidylique:acide carboxylique insaturé $\alpha,\beta$:amine aromatique secondaire se situe dans la plage d'environ 1:0,1:2 à environ 1:2,09:0,01.

**8.** Accélérateur pour un système de résine réactive selon l'une quelconque des revendications 1 à 7 comportant la formule (I) :

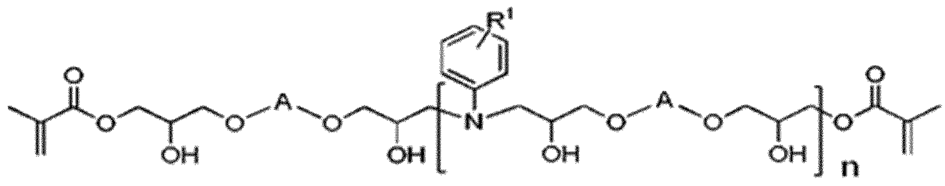

(I),

où

aucun, un ou plusieurs $R^1$ comme substituants sont présents sur l'anneau phényle ;
$R^1$ est choisi dans le groupe constitué d'un halogène, pseudohalogène, alkyle en $C_1$-$C_{20}$, alkyle hydroxy en $C_1$-$C_{20}$, alcényle en $C_2$-$C_{20}$, alcényle hydroxy en $C_2$-$C_{20}$, alcynyle en $C_2$-$C_{20}$, alcynyle hydroxy en $C_2$-$C_{20}$ et phényle ;
n est un nombre entier de 1 à 20, de préférence de 1 à 7, de manière particulièrement préférée de 1 à 3 ; et
A est le résidu du diol qui est contenu dans l'éther diglycidylique (2) ;

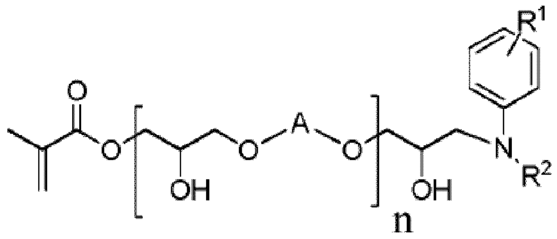

(II),

où

aucun, un ou plusieurs $R^1$ comme substituants sont présents sur l'anneau phényle ;
$R^1$ est choisi dans le groupe constitué d'un halogène, pseudohalogène, alkyle en $C_1$-$C_{20}$, alkyle hydroxy en $C_1$-$C_{20}$, alcényle en $C_2$-$C_{20}$, alcényle hydroxy en $C_2$-$C_{20}$, alcynyle en $C_2$-$C_{20}$, alcynyle hydroxy en $C_2$-$C_{20}$ et phényle ;
$R^2$ est choisi dans le groupe constitué d'un alkyle en $C_1$-$C_{20}$, alkyle hydroxy en $C_1$-$C_{20}$, alcényle en $C_2$-$C_{20}$, alcényle hydroxy en $C_2$-$C_{20}$, alcynyle en $C_2$-$C_{20}$, alcynyle hydroxy en $C_2$-$C_{20}$ et aryle ;
n est un nombre entier de 1 à 20, de préférence de 1 à 5, de manière particulièrement préférée de 1 à 1,5 ; et
A est le résidu du diol qui est contenu dans l'éther diglycidylique (2).

**9.** Accélérateur pour un système de résine réactive selon la revendication 8, où $R^1$ est choisi dans le groupe constitué d'halogène, alkyle hydroxy en $C_1$-$C_{20}$ et alkyle $C_1$-$C_{20}$.

**10.** Accélérateur pour un système de résine réactive selon la revendication 9, où $R^1$ est choisi dans le groupe constitué de chlore, brome et alkyle Ci-Ce.

**11.** Accélérateur pour un système de résine réactive selon l'une quelconque des revendications 1 à 10, où l'éther diglycidylique est un éther diglycidylique de diols choisis dans le groupe constitué de bisphénols, en particulier bisphénol A, bisphénol F et bisphénol S, néopentylglycol, éthylène glycol, résine novolaque phénolique, résine novolaque crésolique et butanediol-1,4.

**12.** Résine réactive, contenant un accélérateur selon l'une quelconque des revendications 1 à 11.

**13.** Composant de résine réactive (A) pour un système de résine réactive, contenant une résine réactive selon la revendication 12.

**14.** Système de résine réactive comportant un composant de résine réactive (A) selon la revendication 13 et un composant durcisseur (B), lequel contient un initiateur.

**15.** Système de résine réactive selon la revendication 14, dans lequel au moins l'un des composants (A) ou (B) contient

une charge inorganique.

**16.** Système de résine réactive selon l'une des revendications 14 ou 15, dans lequel le composant de résine réactive (A) contient :

- au moins un accélérateur selon l'une quelconque des revendications 1 à 11 ;
- au moins une résine d'ossature ;
- au moins un diluant réactif ;
- au moins un inhibiteur ;
- au moins un composé inorganique à prise hydraulique ou polycondensable ; et
- au moins un agent thixotrope,

et le composant durcisseur (B) contient :

- au moins un initiateur pour l'initiation de la polymérisation de la résine réactive ;
- au moins une charge ; et
- de l'eau.

**17.** Utilisation d'un système de résine réactive selon l'une des revendications 14 à 16, pour la fixation chimique de moyens d'ancrage dans des puits ou pour le collage structurel.

**18.** Utilisation d'un accélérateur comme dans l'une des revendications 1 à 11, défini comme accélérateur dans une résine réactive.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 2012164020 A1
- WO 12164020 A1
- EP 1935860 A1
- DE 19531649 A1
- WO 10108939 A1
- US 3297745 A
- US 3772404 A
- US 4618658 A
- GB 2217722 A1
- DE 3744390 A1
- DE 4131457 A1
- US 2011071234 A1
- DE 2312559 A1
- US 3629187 A
- EP 44352 A1
- DE 19902685 A1
- EP 0684906 A1
- DE 4111828 A1
- DE 19961342 A1
- WO 2010108939 A1
- DE 102011077248 B1
- DE 19956509 A1
- US 20020091214 A1
- WO 2002079341 A1
- WO 2002079293 A1
- WO 2011128061 A1
- DE 102009059210 A1
- DE 4231161 A1
- EP 0713015 A1
- WO 12164020 A

### In der Beschreibung aufgeführte Nicht-Patentliteratur

- **M. MALIK et al.** *J. M. S. - Rev. Macromol. Chem. Phys,* 2000, vol. C40, 139-165